# EUROPEAN PATENT SPECIFICATION

(11) **EP 4 225 134 B1**
(45) Date of publication and mention of the grant of the patent: **31.12.2025**
(21) Application number: 21805813.9
(22) Date of filing: 12.10.2021
(51) Int. Cl.: A61B 5/00

(54) **GUIDE WIRE WITH CONDUCTIVE ELEMENT**
FÜHRUNGSDRAHT MIT LEITENDEM ELEMENT
FIL-GUIDE À ÉLÉMENT CONDUCTEUR

(30) Priority: 12.10.2020 US 202063090487 P
(43) Date of publication of application: 16.08.2023
(73) Proprietor: ASAHI INTECC CO., LTD., Seto-shi, Aichi 489-0071 (JP); PATHWAYS MEDICAL CORPORATION, Santa Clara, CA 95054 (US)
(72) Inventor: OGAWA, Minoru, Seto-shi Aichi 489-0071 (JP); HOULE, Philip R., Sunnyvale, California 94087 (US); PATIL, Nitin, Danville, California 94526 (US)
(74) Representative: Winter, Brandl - Partnerschaft mbB
(86) International application number: PCT/US2021/071826
(87) International publication number: WO 2022/082173

(56) References cited:
- EP-A2- 0 925 803
- WO-A1-2015/113044
- WO-A1-2018/017731
- US-A1- 2013 096 455

## Description

### TECHNICAL FIELD

The present invention relates to a guide wire having a sensor. In particular, the present invention relates to a guide wire incorporating a pressure sensor within or along the main body of the guide wire.

### BACKGROUND ART

The guide wire can have a large number of sensors or a sensor assembly incorporated directly into the guide wire (cf. for example US 2013 / 096 455 A1). A guide wire including such sensors may be adapted to measure various physiological parameters in a patient's body. For example, the sensor typically has one or more cables that are passed through the guide wire to electrically combine a sensor element to an electronic assembly. Moreover, WO 2015 / 113 044 A1 relates to methods and apparatus for the assembly and construction of conducting elements in the form of wires or flexible cables for incorporation in space challenged applications.

Generally, the guide wire is composed of a hypotube of a core wire capable of extending along a length or a partial length of the guide wire, and a coiled segment. The guide wire core may be made of stainless steel or Nitinol, and the coiled segment may be made of a wire or a braid that provides flexibility, pushability, and kink resistance to the guide wire. A Nitinol wire can be used by itself or in combination with a stainless steel to further help to improve flexibility and to allow the wire to return to an original shape.

Additionally, a standard diameter of the guide wire is 0.014 inches (0.356 mm), and as a result, accommodation of certain types of sensors or incorporation of plural sensors may be limited due to a relatively small space provided by the guide wire. Furthermore, the guide wire is typically used so as to be inserted and advanced into blood vessels having very tortuous pathways. Thus, the guide wire, and the sensors and electrodes along the guide wire may receive a relatively high stress when the guide wire is pushed, pulled, and twisted on a pathway having many curves and bends.

A guide wire incorporating one or more electrodes along a length of the guide wire may cause additional problems in a structure and use of the guide wire. For example, the presence of plural electrodes along the guide wire may require additional conductive wiring to be passed along the length of the guide wire. Since the guide wire requires a limited space and flexibility, it is desirable that the sensors and/or electrodes arranged along the length of the guide wire are configured so as to meet the limited space and flexibility.

As a result, it is required to design a guide wire, which provides an effective construction of the guide wire incorporating one or more electrodes and/or sensors along the length of the guide wire.

### SUMMARY OF INVENTION

This object is solved by the subject-matter of the independent claim. Further aspects are disclosed in the dependent claims. The invention is set out in the appended set of claims. The present disclosure provides a guide wire including a guide wire core comprising a large-diameter portion, a small-diameter portion located distal to the large-diameter portion and having an outer dimension that is smaller than an outer dimension of the large-diameter portion, and a tapered portion located between the large-diameter portion and the small-diameter portion, a first insulating layer provided on a surface of the guide wire core, and plural conductive traces provided spaced from each other in a lateral direction of the guide wire core on a surface of the first insulating layer, wherein a width dimension and/or a thickness dimension of at least one of the plural conductive traces change on the tapered portion, and in the large-diameter portion and the small-diameter portion, a longitudinal extension of at least one of the plural conductive traces has a sectional area different from those of a longitudinal extensions of the other conductive traces in a transverse-sectional view of the guide wire core.

At least one of the plural conductive traces may have a thickness dimension different from those of the other conductive traces in the transverse-sectional view of the guide wire core at different positions in the length direction.

At least one of the plural conductive traces may have a width dimension different from those of the other conductive traces in the transverse-sectional view of the guide wire core at different positions in the length direction.

A width dimension of at least one of the gaps between the plural conductive traces may be constant in the transverse-sectional view of the guide wire core.

Among the gaps between the plural conductive traces, gaps on a small-outer diameter portion of the guide wire core may have width dimensions larger than those of gaps on a large-outer diameter portion of the guide wire core in the transverse-sectional view of the guide wire core.

Among the gaps between the plural conductive traces, gaps on a small-outer diameter portion of the guide wire core may have width dimensions smaller than those of the gaps on a large-outer diameter portion of the guide wire core in the transverse-sectional view of the guide wire core.

At least one of the plural conductive traces may have a changed shape in the transverse-sectional view of the guide wire core at different positions in the length direction.

The guide wire core includes a large-diameter portion on a butt side, a small-diameter portion located on a front end side of the large-diameter portion, and a tapered portion located between the large-diameter portion and the small-diameter portion, wherein width dimension of at least one of the plural conductive traces changes on the tapered portion.

The guide wire core includes the large-diameter portion on the butt side, the small-diameter portion located on the front end side of the large-diameter portion, and the tapered portion located between the large-diameter portion and the small-diameter portion, wherein thickness dimension of at least one of the plural conductive traces changes on the tapered portion.

The guide wire core includes the large-diameter portion on the butt side, the small-diameter portion located on the front end side of the large-diameter portion, and the tapered portion located between the large-diameter portion and the small-diameter portion, wherein width dimension of at least one of the plural conductive traces may change on the small-diameter portion in the transverse-sectional view of the guide wire core at different positions in the length direction.

The guide wire core includes the large-diameter portion on the butt side, the small-diameter portion located on the front end side of the large-diameter portion, and the tapered portion located between the large-diameter portion and the small-diameter portion, wherein thickness dimension of at least one of the plural conductive traces may change on the small-diameter portion in the transverse-sectional view of the guide wire core.

Each of the plural conductive traces has each electrical connection portion, and the plural electrical connection portions may be arranged on one straight line of the guide wire core.

Each of the plural conductive traces has each electrical connection portion. Among the plural electrical connection portions, first plural electrical connection portions and second plural electrical connection portions may be arranged in parallel along the length direction of the guide wire core.

The guide wire core has a flat attachment portion, each of the plural conductive traces has each electrical connection portion, and at least some of the plural electrical connection portions may be disposed on the attachment portion.

The guide wire core has the flat attachment portion, each of the plural conductive traces has each electrical connection portion, at least some of the plural electrical connection portions may be disposed on the attachment portion, and the electrical connection portions disposed on the attachment portion may be arranged on one straight line along the length direction of the guide wire core.

The guide wire core has the flat attachment portion, each of the plural conductive traces has each electrical connection portion. Among the plural electrical connection portions, the first plural electrical connection portions and the second plural electrical connection portions may be arranged in parallel on the attachment portion.

A second insulating layer that covers the first insulating layer and the plural conductive traces is provided, and the first insulating layer may be made of a material having a lower dielectric constant than of the second insulating layer.

The second insulating layer that covers the first insulating layer and the plural conductive traces is provided, and the first insulating layer may be made of a material having a higher adhesiveness with the surface of the guide wire core than of the second insulating layer.

The second insulating layer that covers the first insulating layer and the plural conductive traces is provided, and the second insulating layer may be made of a material having a higher moisture resistance than of the first insulating layer.

A metal layer made of a material having a higher conductivity than of a material for the guide wire core may be disposed on the surface of the guide wire core.

The present disclosure is also applied to a long medical equipment including any of the aforementioned guide wires.

Yet another aspect of the present disclosure executes a step of providing a guide wire core, a step of forming a first insulating layer on a surface of the guide wire core, a step of forming plural conductive traces spaced from each other in a lateral direction of the guide wire core on a surface of the first insulating layer in which the plural conductive traces have different sectional areas in a transverse-sectional view of the guide wire core, and a step of forming a second insulating layer that covers the first insulating layer and the plural conductive traces.

The guide wire can incorporate a large number of different sensors within or along a main body of the guide wire. In a certain modification example, optionally, a pressure sensor having one or more electrodes may be incorporated in the guide wire along the main body of the guide wire or on the distal end of the guide wire. The guide wire having one or more electrodes directly integrated along the main body of the guide wire may have a proximal coil attached to an electrode assembly having one or more electrodes, and a distal coil attached to a distal end of the electrode assembly. The guide wire core may extend through the guide wire assembly in the length direction, or may extend partially or thoroughly through the electrode assembly.

A modification example for assembling a guide wire assembly may generally include a step of providing a core wire having a tapered distal portion, a step of fixing one or more conductive wires to the core wire by passing the core wire through a wire receiving channel demarcated via or along a sensor package, and then a step of sealing the one or more conductive wires and the core wire.

An example of a method for forming the guide wire assembly may generally include a step of providing the guide wire core, a step of disposing an insulating layer on the surface of the guide wire core, and a step of printing one or more conductive traces directly on the surface of the insulating layer.

Another example of the method for forming the guide wire assembly may generally include a step of providing the guide wire core, a step of disposing the insulating layer on the surface of the guide wire core, and a step of disposing an aerosolized conductive ink on the surface of the insulating layer to form one or more conductive traces.

Yet another example of the method for forming the guide wire assembly may generally include a step of providing the guide wire core, a step of disposing the insulating layer on the surface of the guide wire core, and a step of removing a part of the conductive layer such that one or more conductive traces are formed on the insulating layer.

In a modification example, when forming the guide wire assembly, generally, a pressure sensor packaging may include a sensor casing constituting a cylindrical housing that surrounds or supports constituents of the pressure sensor fixed therein. In the sensor casing, a detection window may be demarcated along a lateral face of the casing, and this detection window allow the internal pressure sensor to be exposed to a fluid environment. A sensor core is fixed within the sensor casing and connected to a flex circuit extending from a proximal end of the sensor casing, and, in some cases, connected to a controller or processor via one or more lead wires extending along the length of the guide wire. The conductive traces or wires along the flex circuit may be attached directly to one or more corresponding conductive wires extending toward the proximal side through the guide wire main body, so that the conductive traces or wires are electrically connected to the controller or processor.

Another modification example includes a configuration in which the flex circuit extends toward the proximal direction from the sensor casing, but the flex circuit may be electrically connected to one or more conductive ring elements instead of being directly attached to one or more conductive wires. The ring elements are electrically connected to one or more conductive wires. The ring elements are arranged coaxially with and adjacent to each other, and the number of the elements used may depend on the number of required electrical connections. One or more conductive wires may be selectively and electrically combined with a specific pad or trace of the flex circuit, such that each ring element is electrically connected to a single pad or trace. Subsequently, each ring element may be selectively and electrically combined with the conductive wire along an inner diameter of the ring element, and the remainder of the ring element can be electrically connected to another conductor or component as needed.

The sensor casing may demarcate a longitudinal passage penetrating the whole casing to allow the guide wire core to pass therethrough. Furthermore, the casing may demarcate a distal opening portion on which the front end of the guide wire is positioned and fixed so as to extend from the distal end of the casing, and the core of the guide wire longitudinally extends through the casing adjacent to or below the flex circuit, the pressure sensor, and the detection window. The sensor core is shown to be fixed within the casing adjacent to the flex circuit proximally extending from the casing.

In yet another modification example for electrically combining elements within or along the guide wire, the guide wire assembly may have a conductive ink printed on a polymer substrate to form a subassembly for transmitting signals from one end of the guide wire or catheter to the other end. The conductive traces are used directly on a device substrate, and then the traces are insulated by a dielectric material, so that necessity of the conductive wire, and relevant treatment and handling can be eliminated.

A polymer layer (e.g., PET, PTFE, etc.) may be coated over the core of the guide wire via heat shrink to provide an insulating substrate. The polymer layer may be coated or laid over the whole guide wire core, or a part of the distal end of the guide wire core may be left non-coated in order to fix a pressure sensor assembly. Subsequently, one or more conductive traces (e.g., nanosilver, nanogold, nanocopper, etc.) may be printed directly on the polymer layer such that the traces extend from one or more corresponding distal pads to one or more corresponding proximal pads.

The one or more conductive traces are printed directly on the polymer layer and therefore can be configured in a large number of various patterns. Once one or more conductive traces are printed on the polymer layer, the traces may be subsequently insulated. In one variation for insulating the trace, the edge of the trace that need to be left exposed for forming the electrical connection pad is masked, and then another polymer layer is deposited over the conductive trace. For example, another polymer layer (PTFE, Paralin, etc.) can be deposited on the exposed conductive trace using another heat shrinkable tube and layer, or using a physical vapor growth method, a dip coating method, or the like.

In yet another variation, a conductive coating can be provided on the dielectric layer by a bulk metallization process such as physical vapor growth deposition (PVD) or by electroplating, electroless plating, a method of printing a broader metal layer on a dielectric layer using conductive inks, or the like. Such a metal layer can eliminate or reduce noises and improve Signal to Noise Ratio (SNR) of the system by providing an EM shield.

As another variation for insulating the traces, a dielectric polymer is printed directly on the conductive traces using a polymer ink. When the dielectric polymer is printed directly on the conductive traces using the polymer ink, in a printing process, the polymer ink is selectively printed to form an insulating layer, meanwhile the polymer ink can be used to form the conductive pad for electrically combining with components by exposing a part of the conductive traces.

Regardless of which method is used, the resulting guide wire core and polymer layer may be combined with the pressure sensor assembly. One or more ring elements may be, along a part of their inner diameter, electrically combined with corresponding pads exposed along the flex circuit. A second portion along the one or more ring elements may be electrically combined with the corresponding pads of the conductive traces disposed on the polymer layer to electrically combine with the pressure sensor assembly (or any other component). Subsequently, the distal coil tip may be attached to the distal end of the sensor casing to reflow or mold the polymer on the guide wire core along the center portion, the distal coil or tip along the distal portion, and the remainder of the guide wire core along the proximal portion, as well as (if used) a portion between the electrodes.

Another modification example of the assembly method includes a polymer layer separately formed prior to being disposed on the guide wire core. The conductive traces may be printed directly on the outer layer of the polymer, together with their corresponding exposed pads extending over the length of the polymer layer. Similarly, the insulating layer may also be printed directly on the conductive traces. Using a previously printed polymer layer, the guide wire core may be inserted into the polymer layer and bonded to the polymer layer with any number of suitable adhesives, e.g. cyanoacrylate. Subsequently, the pressure sensor assembly is fixed to the guide wire core, and the flex circuit may be directly and electrically combined with the exposed pads of the attachment to complete the electrical connection. In another variation for printing the conductive traces, a polymer tube may be disposed on the guide wire core to print one or more conductive traces on the outer layer of the tube. Subsequently, circular rings may be printed on the polymer tube using the conductive ink such that the rings coincide with exposed regions of the conductive traces, and the flex circuit and other components of the pressure sensor assembly can electrically combine with the conductive traces via the connection to the circular rings. Since the circular rings are printed in a circumferential direction of the tube, the exposed regions may be displaced from each other in the longitudinal direction such that the rings can be printed on the whole circumference of the tube. In addition, preferably, there are sufficient spaces in the longitudinal direction between the exposed regions, so that the rings can be printed coaxially with each other without interfering the rings. In another modification example, partial circumferential rings may be printed rather than entire circumferential ring.

Yet another modification example for producing conductive traces may include a first insulating polymer layer disposed on the outer face of the guide wire core (e.g. PARYLENE (Specialty Coating Systems, Inc., Indianapolis, IN), TEFLON (E. I. Du Pont De Nemours, Wilmington, DE), polyimide, etc.). Subsequently, a second conductive polymer layer containing a conductive material (gold, silver, copper, etc.) may be coated on the first polymer layer using any number of processes, such as electroless deposition, and physical vapor growth. The thickness of the conductive layer depends on the application and is often determined in consideration of both electrical requirements (current-carrying capacity) and mechanical requirements (rigidity, etc.) of the device. This second conductive layer may be separated into individual conductive elements using laser microfabrication, photochemical etching, or the like.

Subsequently, the whole assembly can be insulated by using a dielectric insulating polymer, in a form of either coating or heat shrink (Teflon, PET, etc.), depending on the application. Depending on the application, a plurality of individual conductive elements can be formed. In addition, depending on the application, both ends of connection terminals can be formed into various sizes and shapes to facilitate connection with the individual conductive elements formed. Since such a structural technique makes it possible to directly form a plurality of individual conductive elements on the device, it is not required to remove materials for the purpose of accommodating individual conductive wires, or to hollow the device for the purpose of accommodating the conductive wires and elements. Thus, the performance of the intended device is significantly improved and the manufacturing cost is reduced.

### BRIEF DESCRIPTION OF DRAWINGS

FIG. 1A is a front view of a guide wire having a sensor.
FIG. 1B is a front view of a guide wire core to which the sensor is attached.
FIG. 2 is an enlarged view of the sensor.
FIG. 3 is a perspective view of the sensor.
FIG. 4 is a diagram illustrating an arrangement of plural electrical connection portions formed on a distal end side of the guide wire.
FIG. 5 is a diagram illustrating an arrangement of the plural electrical connection portions formed on a proximal end side of the guide wire.
FIG. 6 is a diagram illustrating a connection between a sensor assembly and the plural electrical connection portions formed on the distal end side of the guide wire.
FIG. 7 is a plan view of the sensor assembly.
FIG. 8 is a longitudinal sectional view illustrating a relationship between the sensor assembly and the guide wire.
FIG. 9 is a sectional view viewed from a direction of arrows IX-IX in FIG. 8.
FIG. 10 is a sectional view viewed from a direction of arrows X-X in FIG. 8.
FIG. 11 is a transverse-sectional view illustrating a modification example of FIG. 10.
FIG. 12A is a transverse-sectional view illustrating a modification example of an insulating layer that covers the guide wire.
FIG. 12B is a transverse-sectional view illustrating another modification example of the insulating layer that covers the guide wire.
FIG 13 is a diagram illustrating an example that plural conductive traces are formed in a straight shape.
FIG 14 is a diagram illustrating an example that the plural conductive traces are formed in a spiral shape.
FIG. 15 is a diagram illustrating an example that widths of the plural conductive traces are varied in a longitudinal direction.
FIG. 16 is an enlarged view of the distal end in FIG. 15.
FIG. 17 is a diagram viewed from a direction of the arrow in FIG. 16.
FIG. 18 is a diagram illustrating a modification example of FIG. 17.
FIG. 19 is a diagram illustrating an example that the sensor is mounted on the guide wire.
FIG. 20 is a diagram illustrating another example that the sensor is mounted on the guide wire.
FIG. 21 is a perspective view illustrating a flat distal end of the guide wire.
FIG. 22 is a plan view of the flat distal end of the guide wire.
FIG. 23 is a diagram illustrating a state that the sensor is attached to the flat distal end of the guide wire.
FIG. 24 is a front view of the flat distal end of the guide wire.
FIG. 25 is a longitudinal sectional view illustrating an example that the sensor is mounted on the guide wire.
FIG. 26 is a longitudinal sectional view illustrating another example that the sensor is mounted on the guide wire.
FIG. 27 is a sectional view illustrating yet another example that the sensor is mounted on the guide wire.
FIG. 28 is an enlarged longitudinal sectional view of a part to which the sensor is attached.
FIG. 29 is a longitudinal sectional view illustrating an example that plural sensors are mounted on the guide wire.
FIG. 30 is a longitudinal sectional view illustrating a structural example on a surface side of the guide wire.
FIG. 31 is a longitudinal sectional view illustrating another structural example on the surface side of the guide wire.
FIG. 32 is a longitudinal sectional view illustrating yet another structural example on the surface side of the guide wire.
FIG. 33A is a transverse-sectional view of the guide wire in which plural conductive layers are formed in a build-up manner.
FIG. 33B is a transverse-sectional view of another guide wire in which plural conductive layers are formed in a build-up manner.
FIG. 34 is a longitudinal sectional view of the guide wire, illustrating a relationship between inner conductive traces and outer ring electrodes.
FIG. 35 is a sectional view taken along line L1 in FIG. 34.
FIG. 36 is a sectional view taken along line L2 in FIG. 34.
FIGS. 37A-37F are sectional views illustrating the relationship between the conductive traces and the ring electrodes, in an order from the distal end of the guide wire.
FIG. 38 is a longitudinal sectional view illustrating an example that the guide wire core is used as an electrical wiring (e.g. ground electrode).
FIG. 39 is a longitudinal sectional view illustrating another example that the guide wire core is used as a ground electrode.
FIG. 40 is a schematic diagram illustrating an example of an electrical connection of the guide wire.
FIG. 41 is a schematic diagram illustrating another example of the electrical connection of the guide wire.
FIG. 42 is a schematic diagram illustrating yet another example of the electrical connection of the guide wire.
FIG. 43 is a schematic diagram illustrating another example of the electrical connection of the guide wire.
FIG. 44 is a diagram illustrating an example that plural conductive traces are deployed into a plane.
FIGS. 45A-45G are diagrams illustrating examples of a manufacture method of the guide wire.
FIG. 46 is a diagram following FIG. 45.

### DESCRIPTION OF EMBODIMENTS

In the present disclosure, plural conductive traces can be formed on at least a part of a longitudinal region of a guide wire. The plural conductive traces are electrically connected to at least one sensor provided on the guide wire. The sensor measures e.g. parameters such as a pressure, a temperature, and a flow rate of a body tissue into which the guide wire is inserted. The sensor physically or chemically measures these parameters or other parameters. Signals measured by the sensor are output to a measuring device disposed outside the guide wire via the conductive traces.

In the present disclosure, for example, a guide wire will be explained as a long medical equipment. However, the present disclosure can be applied not only to guide wires but also catheters. The present disclosure can also be applied to e.g. balloon catheters, microcatheters, cardiac catheters, pulmonary artery catheters, angiographic catheters, urinary catheters, gastrointestinal catheters, or the like.

In addition to the embodiments described below, various modification examples are included in the present disclosure. It would be possible to replace a part of the configurations described in an embodiment with a configuration described in another embodiment. It would be possible to add a configuration of another embodiment to a configuration of an embodiment.

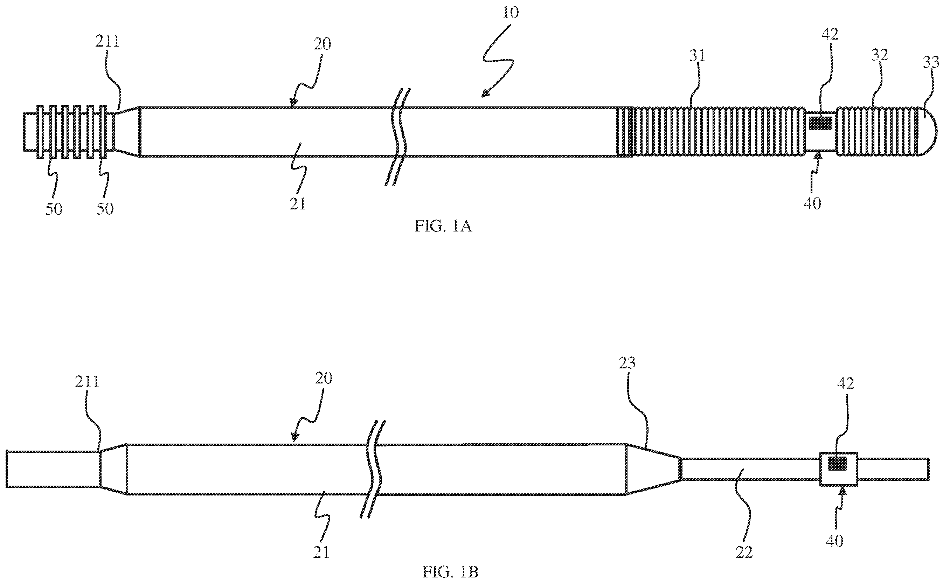

FIG. 1A illustrates a whole of a guide wire 10 in which a sensor 42 is attached to a conductive trace. FIG. 1B is a front view of a guide wire core 20 to which the sensor 42 is attached. The guide wire 10 includes e.g. the guide wire core 20 and a sensor assembly 40 provided on a front end side of the guide wire core 20. In the present disclosure, a base end side of the guide wire 10 is referred to as a proximal end side or a butt side, and a front end side of the guide wire 10 is referred to as a distal end side in some cases. The guide wire 10 is formed by assembling coil bodies 31 and 32 and respective ring electrodes 50 onto the guide wire core 20 illustrated in FIG. 1B. The coil bodies 31 and 32 are fixed to a small-diameter portion 22 of the guide wire core 20 using a fixing material. A front end tip 33 located on the front end of the guide wire core 20 is formed in an almost hemispherical shape by a fixation member that fixes the front end of the small-diameter portion 22 of the guide wire core 20 with the front end of the coil body 32. For example, a brazing material or an adhesive can be used for the fixing material.

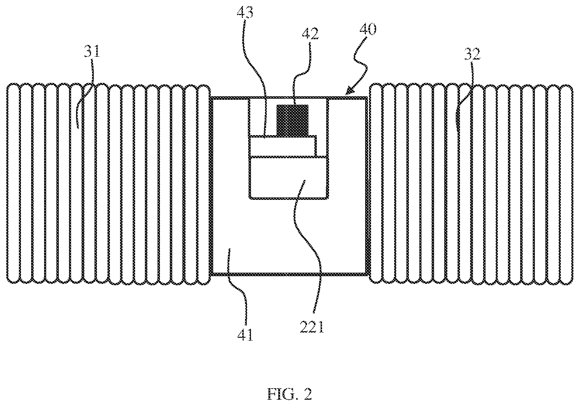

The guide wire core 20 is made of e.g. Nitinol or stainless steel. The guide wire core 20 includes a large-diameter portion 21 on the butt side, the small-diameter portion 22 located on the front end side of the large-diameter portion 21, and a tapered portion 23 located between the large-diameter portion 21 and the small-diameter portion 22. On the distal end side of the small-diameter portion 22, a sensor attachment portion 221 is formed as illustrated in FIG. 2. An external connection portion 211 is formed on the proximal end side of the large-diameter portion 21. The external connection portion 211 has the plural ring electrodes 50 as an example of "parts that electrically connect the guide wire to an external circuit.

The tapered portion 23 is formed so as to gradually decrease in diameter such that smooth connection from the distal end side of the large-diameter portion 21 to the proximal end side of the small-diameter portion 22 is achieved. The plural coil bodies 31 and 32 are provided outside the small-diameter portion 22. The sensor assembly 40 is disposed between the coil body 31 on the proximal end side and the coil body 32 on the distal end side. The coil bodies 31 and 32 are made of e.g. stainless steel, platinum (Pt), a platinum-iridium alloy (Pt/Ir), or the like. As described below, the guide wire core 20 may have one coil body. Another example of arrangement for the sensor assembly 40 will be described below.

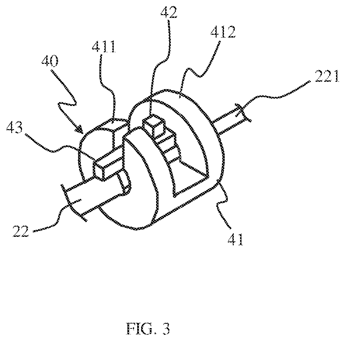

FIG. 2 is an enlarged view of the sensor assembly 40. FIG. 3 is a perspective view of the sensor assembly 40. The sensor assembly 40 includes e.g. a sensor housing 41, the sensor 42, and a wiring portion 43. The sensor housing 41 is provided outside the sensor attachment portion 221.

The sensor housing 41 is formed in an almost cylindrical shape in which a central portion in the longitudinal direction is opened. The sensor attachment portion 221 of the guide wire core 20 is provided so as to penetrate the sensor housing 41 in the longitudinal direction. As described below, plural conductive traces 25 are formed spaced from each other in the lateral direction on the outside of the guide wire core 20 from the sensor attachment portion 221 to the external connection portion 211. The plural conductive traces 25 include electrical connection portions on both the sensor attachment portion 221 and the external connection portion 211. The lateral direction of the guide wire core 20 refers to e.g. a circumferential direction of the guide wire core 20. The cross section of the guide wire core 20 is not only the circular shape but also an oval or polygonal shape.

Although the present disclosure will be explained with reference to a case that five conductive traces 25 are provided, the number of conductive traces 25 only needs to be two or more. Conductive traces used for shielding lines or the like can be provided as needed.

The sensor 42 is attached to the outside of the sensor attachment portion 221 by the wiring portion 43. Each electrical terminals of the sensor 42 is electrically connected to the corresponding conductive trace 25 among the plural conductive traces 25 via the wiring portion 43. The wiring portion 43 is formed e.g. as an interposer substrate. The wiring portion 43 may be attached to the sensor attachment portion 221 directly or via a flexible wiring board.

The sensor housing 41 prevents a pressure of a blood or the like from being applied to the sensor 42 and the wiring portion 43 from the longitudinal direction of the guide wire core 20 on a wall portion 412 on the distal end side. A wall portion 411 on the proximal end side of the sensor housing 41 is formed in an almost U-shape or C-shape in a transverse-sectional view, and pinches the wiring portion 43 from both sides in the width direction to support the wiring portion 43. Thereby, the sensor housing 41 firmly holds the sensor 42 and the wiring portion 43 to suppress a relative displacement with respect to the small-diameter portion 22.

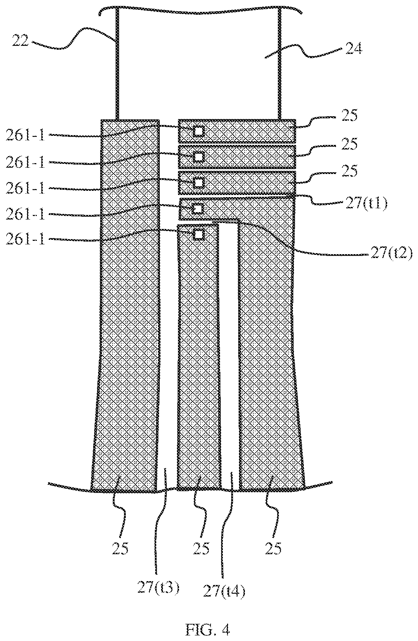

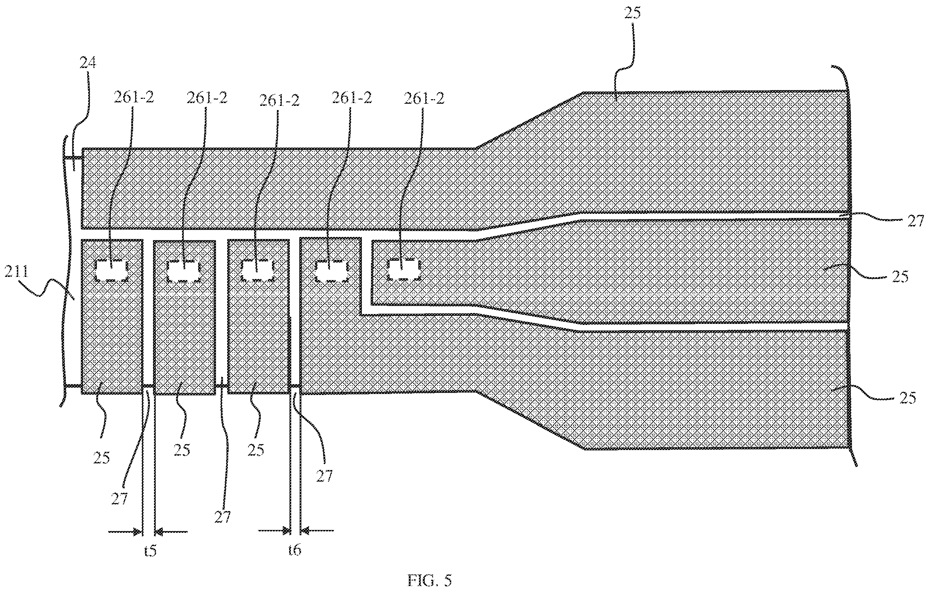

FIG. 4 illustrates an arrangement of plural electrical connection portions 261-1 formed on the distal end of the guide wire. FIG. 5 illustrates an arrangement of plural electrical connection portions 261-2 formed on a proximal end side of the guide wire core 20. A first insulating layer 24 is provided on the surface of the guide wire core 20. On the surface of the first insulating layer 24, plural conductive traces 25 are formed spaced from each other in a lateral direction of the guide wire core 20. Gaps 27 are formed between the conductive traces 25 adjacent to each other. To form the gaps 27, e.g. the conductive layer formed on the surface of the first insulating layer 24 is etched into a predetermined shape by a laser beam or the like. The gaps 27 having a predetermined width dimension can be formed by controlling an output and/or scanning trajectory, or the like of the laser beam. Ordinarily, the gaps 27 are filled with an insulating material. The gaps 27 can also be referred to as insulating space segments.

The plural conductive traces 25 on the distal end side have electrical connection portions for the connection with the wiring portion 43. To form the electrical connection portions, predetermined portions of a second insulating layer 26 that covers the surface sides of the respective conductive traces 25 are opened, and the respective conductive traces 25 are exposed, as described below. That means, the opening portions provided on the insulating layer that covers the surface of the guide wire core 20 for the purpose of enabling electrical connection between the conductive traces 25 and the wiring portion 43 are referred to as electrical connection portions in the present disclosure. In the present disclosure, the opening portions 261-1 formed on predetermined portions of the insulating layer 26 are referred to as electrical connection portions 261-1 for convenience sake in some cases.

The plural conductive traces 25 on the proximal end side also have the electrical connection portions 261-2. The plural conductive traces 25 on the proximal end side are electrically connected to the ring electrodes 50 or second conductive traces 51 described below.

As described below, when other conductive traces 51 are provided outside the conductive traces 25 while interposing the second insulating layer 26 therebetween, predetermined portions of a third insulating layer 28 that covers the other conductive traces 51 are opened to form electrical connection portions. The electrical connection portions can be rephrased as opening portions, i.e. via holes, which are formed at predetermined positions on the insulating layer for the purpose of the electrical connection.

The width dimensions of the respective gaps 27 may be set to the same value or to different values. For example, as illustrated in FIG. 4, width dimensions t3 and t4 of the gaps 27 formed parallel to the longitudinal direction of the guide wire core 20 can be set to be larger than width dimensions t1 and t2 of the gaps 27 formed orthogonal to the longitudinal direction of the guide wire core 20. In FIG. 4, the width dimensions of the gaps 27 are appended in parentheses accompanying the symbol 27.

In the example of FIG. 5, the guide wire core 20 made of a conductive material such as stainless steel is used as an electrical ground. If the guide wire core 20 is not used as the electrical ground, any of the plural conductive traces 25 can be used as the electrical ground. The guide wire core 20 and any one of the conductive traces 25 can also be used as the electrical ground.

In the present disclosure, a width dimension t6 of at least one of the gaps 27 between the plural conductive traces 25 formed spaced from each other in the lateral direction of the guide wire core 20 can be different from width dimensions t5 of the other gaps 27. Thereby, an electrical effect such as noise reduction can be provided to at least one conductive trace 25. For example, a gap between the conductive trace used as a signal wire and the conductive trace used as the ground wire can be narrowed to reduce noises generated in the signal. In a case that the guide wire core 20 used as the ground is connected with the ring electrodes 50 via the via holes, a via diameter is larger than a via diameter in a case that only the second insulating layer 26 is opened and the via holes are connected to the conductive traces 25. Thus, the width dimensions of the gaps 27 corresponding to these via holes are different from each other.

The electrical connection portions 261-1 of the respective conductive traces 25 on the distal end side are arranged on one straight line in the longitudinal direction of the guide wire core 20, as illustrated in FIG. 4. Thereby, the electrical connection portions 261-1 can be easily connected with plural pads 431 arranged on one straight line on the wiring portion 43, as described below. The electrical connection portions 261-2 of the respective conductive traces 25 on the proximal end side are also arranged on one straight line in the longitudinal direction of the guide wire core 20, as illustrated in FIG. 5.

As described below, a transmission property suitable for a role of the respective conductive traces 25 can be achieved by varying the width dimensions of the respective conductive traces 25.

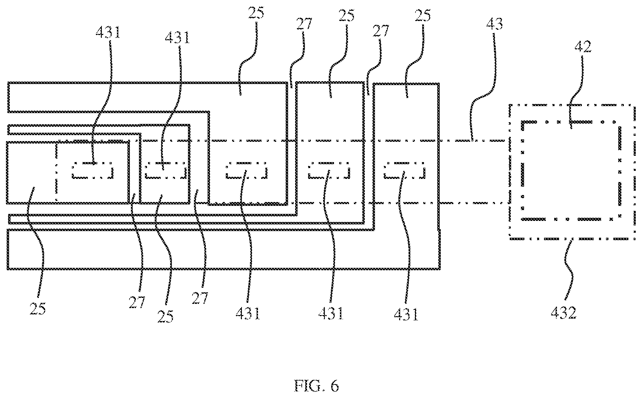

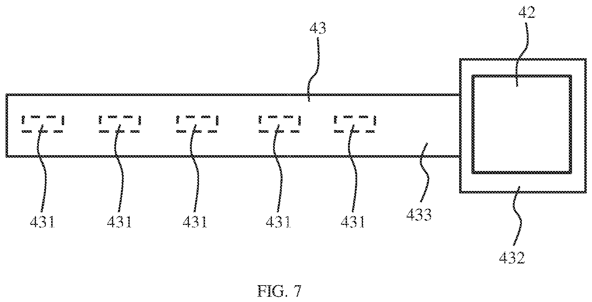

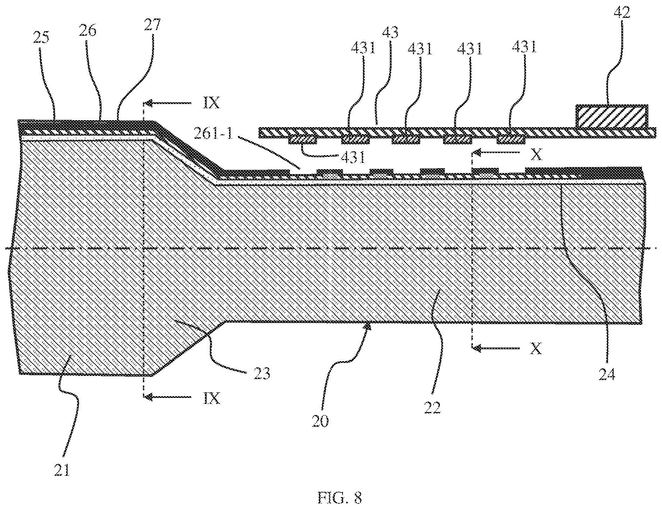

FIG. 6 illustrates an arrangement relationship between the wiring portion 43 and the respective conductive traces 25 formed on the distal end side of the guide wire core 20. FIG. 7 is a plan view of the sensor assembly 40. FIG. 8 is a longitudinal sectional view illustrating a relationship between the sensor assembly 40 and the guide wire core 20. In FIG. 7 and FIG. 8, the sensor housing 41 is omitted. In FIG. 8, the insulating layers 24 and 26 and the conductive traces 25 are illustrated only on the upper half of the guide wire core 20.

The wiring portion 43 includes a sensor connection portion 432 and a pad-formed portion 433 extending from the sensor connection portion 432 toward the proximal end side. The plural pads 431 are arranged on the one straight line on the guide wire core 20 side of both sides of the pad-formed portion 433. The interval of the formed plural pads 431 coincide with the interval of the formed electrical connection portions of the plural conductive traces 25. Thus, as illustrated in FIG. 8, the sensor 42 can be electrically connected to the respective conductive traces 25 simply by placing the wiring portion 43 on the small-diameter portion 22 of the guide wire core 20. Thereby, the guide wire 10 can be efficiently assembled, and the manufacturing cost can be reduced. In contrast, if the plural electrical connection portions are arranged spaced from each other in the circumferential direction of the small-diameter portion 22 of the guide wire core 20, the operation of attaching the sensor 42 to the guide wire core 20 is complicated. However, in spite of such a characteristic in manufacturing, a configuration in which the electrical connection portions are not arranged on the one straight line is also included in the scope of the present disclosure.

The respective pads 431 and the respective conductive traces 25 can be electrically connected to each other using e.g. a conductive adhesive. Alternatively, each pad 431 is fitted into a corresponding electrical connection portion, so that the sensor 42 and the respective conductive traces 25 can be electrically connected to each other using no conductive adhesive. As illustrated in FIG. 3, the sensor 42 and the wiring portion 43 are protected and positioned by the sensor housing 41. Thus, there is little possibility that a relative displacement is caused in the longitudinal or circumferential direction of the guide wire core 20 between the wiring portion 43 and the small-diameter portion 22 of the guide wire core 20. In other words, each pad 431 of the wiring portion 43 can be fixed and electrically connected to each conductive trace 25 at a predetermined position using no conductive adhesive.

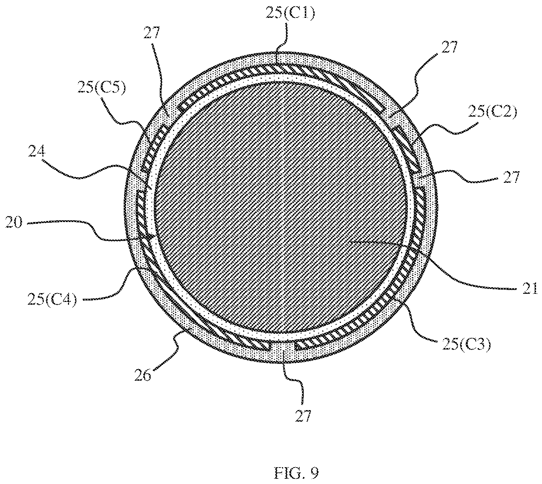

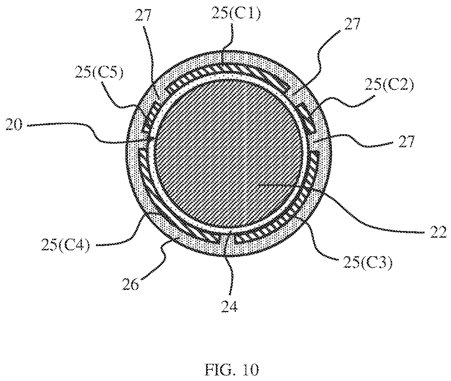

FIG. 9 is a sectional view viewed from a direction of arrow IX in FIG. 8. FIG. 10 is a sectional view viewed from a direction of arrow X in FIG. 8. FIG. 9 is a transverse-sectional view across the large-diameter portion 21 of the guide wire core 20. FIG. 10 is a transverse-sectional view across the small-diameter portion 22 of the guide wire core 20.

The first insulating layer 24 is formed over the whole periphery on the surface of the guide wire core 20. On the surface of the first insulating layer 24, the plural conductive traces 25 spaced from each other via the gaps 27 in the lateral direction of the guide wire core 20 are formed, as described above. The second insulating layer 26 is formed so as to cover both the first insulating layer 24 and the plural conductive traces 25. The first insulating layer 24, the conductive traces 25, and the second insulating layer 26 are formed in a build-up manner. For the first insulating layer 24 and the second insulating layer 26, a material according to properties required for the guide wire 10 can be used. The properties required for these insulating layers 24 and 26 include e.g. electrical insulation, core adhesiveness, dielectric property (low ε), heat resistance, sterilization resistance, scratch resistance, abrasion resistance, chemical resistance, good slidability, water and moisture resistance, rust resistance, adhesiveness with hydrophilic coating agents (hyaluronic acid, silicone, etc.), and the like.

The properties of the first insulating layer 24 can be different from those of the second insulating layer 26. In an example, the first insulating layer 24 may be made of a material having a dielectric constant lower than of the second insulating layer 26. When the dielectric constant of the first insulating layer 24 is decreased, a parasitic capacitance between the conductive traces 25 and the guide wire core 20 can be decreased. That means, when the guide wire core 20 is used as an electrical wiring together with the conductive traces 25, the mutual capacitance between the conductive traces 25 and the guide wire core 20 tends to be significantly higher than the mutual capacitance between the conductive traces. When suppressing increase in this mutual capacitance, it is effective that the first insulating layer 24 sandwiched between the conductive traces 25 and the guide wire core 20 is made of a dielectric material having a lower dielectric constant. In another example, the first insulating layer 24 may be made of a material having a higher adhesiveness with the surface of the guide wire core 20 than of the second insulating layer 26. In yet another example, the second insulating layer 26 may be made of a material having a moisture resistance higher than of the first insulating layer 24.

Examples of the material that can be used for the first insulating layer 24 and/or the second insulating layer 26 include an epoxy resin, a glass epoxy resin, a bismaleimide triazine resin, BCB, polyimide, polyamide, polyamideimide, polyurethane, LCP (liquid crystal polymer), PE (polyethylene), PET (polyethylene terephthalate), PFA (perfluoroalkoxy fluororesin), PTFE (polytetrafluoroethylene), ETFE (copolymer of tetrafluoroethylene (C2F4) and ethylene (C2H4)), PEEK (polyetheretherketone), a parylene resin, solder resist, and the like.

As an example, the first insulating layer 24 may be made of a polyimide, and the second insulating layer 26 may be made of a polyimide (filler-containing reinforced grade). As another example, the first insulating layer 24 may be made of an LCP, and the second insulating layer 26 may be made of a polyimide. As yet another example, the first insulating layer 24 may be made of an LCP, and the second insulating layer 26 may be made of a PEEK. As yet another example, the first insulating layer 24 may be made of a polyimide, and the second insulating layer 26 may be made of a PTFE. As another example, the first insulating layer 24 may be made of a polyimide, and the second insulating layer 26 may be made of a parylene.

In FIG. 9 and FIG. 10, identification numbers such as C1 and C2 are appended in parentheses accompanying the symbol 25 for the purpose of distinguishing the plural conductive traces 25 from each other. When the respective conductive traces 25 (C1) to 25 (C5) are not distinguished, they are collectively referred to as the conductive traces 25. In examples of FIG. 9 and FIG. 10, at least one of the plural conductive traces 25 (C1) to 25 (C5) has a sectional area different from those of the other conductive traces in the transverse-sectional view of the guide wire core 20.

For example, the conductive trace 25 (C1) has a sectional area larger than of the conductive trace 25 (C2) or the conductive trace C25 (C5). The conductive trace 25 (C2) has a sectional area smaller than of the conductive trace 25 (C1), the conductive trace 25 (C3) or the conductive trace 25 (C4). From another viewpoint, there are plural groups with different sectional areas: a first group of the conductive traces 25 (C1), 25 (C3), and 25 (C4) having large sectional areas; and a second group of the conductive traces 25 (C2) and 25 (C5) having small sectional areas.

The sectional area of one conductive trace 25 is determined by multiplying a width dimension and a thickness dimension. Thus, difference in the sectional area of one conductive trace 25 from the sectional areas of the other conductive traces 25 means difference in at least either the width dimension or the thickness dimension.

In both examples of FIG. 9 and FIG. 10, the thickness dimensions of the respective conductive traces 25 are the same, and conductive traces having different width dimensions are mixed together. Since the thickness dimensions of the respective conductive traces 25 are the same, the conductive trace having a longer width dimension has a larger sectional area. The width dimension of the conductive trace 25 is also referred to as a trace width.

When the conductive traces 25 have the same thickness dimensions, the parasitic capacitance (also called stray capacitance.) can be increased by widening the width dimensions of the conductive traces of the power supply system (VCC, GND), and the increase of the parasitic capacitance can decrease the power supply noises. The conductive traces 25 having the small width dimensions may be used as signal wires.

When contrasting FIG. 9 and FIG. 10, the thickness dimensions of the respective conductive traces 25 are the same. However, the respective conductive traces 25 illustrated in FIG. 10 have smaller width dimensions than of the respective conductive traces 25 illustrated in FIG. 9. In examples of FIG. 9 and FIG. 10, the width dimensions of the conductive traces 25 are values corresponding to a ratio between the diameter of the small-diameter portion 22 and the diameter of the large-diameter portion 21. In an aspect of the present disclosure, the width dimensions of the respective conductive traces 25 are set to values corresponding to the variation tendency in the diameter dimension of the guide wire core 20, but the present disclosure is not limited to this setting. The width dimensions of the respective conductive traces 25 may be set to the same value regardless of the variation of the diameter dimension of the guide wire core 20. The width dimensions of the respective conductive traces 25 may be set to values according to an inverse tendency to the variation tendency in the diameter dimension of the guide wire core 20. That means, the present disclosure also includes a configuration in which the width dimensions of the conductive traces 25 on the small-diameter portion 22 are larger than the width dimensions of the conductive traces 25 on the large-diameter portion 21.

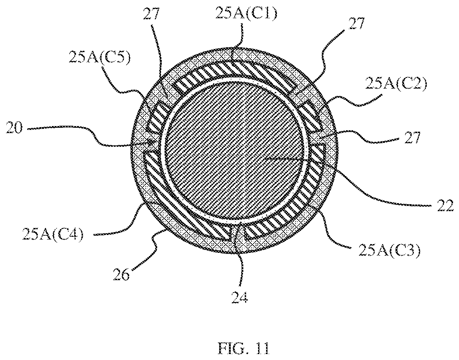

FIG. 11 is a transverse-sectional view illustrating a modification example of FIG. 10. In the following description, alphabetical characters are appended to symbols for distinguishing the configurations of the modification examples. For example, symbol 25A is provided to the conductive traces in FIG. 11. When the conductive traces 25 described in FIG. 1 to FIG. 10 are not distinguished from the conductive traces 25A described in FIG. 11, they are collectively referred to as conductive traces 25. In the example of FIG. 11, the thickness dimensions of the conductive traces 25A (C1) to 25A (C5) are larger than the thickness dimensions of the conductive traces 25 (C1) to 25 (C5) illustrated in FIG. 10. Accordingly, the sectional areas of the conductive traces 25 on the small-diameter portion 22 in the example of FIG. 11 are larger than in the example of FIG. 10.

In FIG. 11, the respective conductive traces 25A (C1) to 25A (C5) have a common thickness dimension, as described for FIG. 9 and FIG. 10. The conductive traces illustrated in FIG. 11 include plural groups with different width dimensions. There are two groups: a first group of the conductive traces 25A (C1), 25A (C3), and 25A (C4) having large sectional areas; and a second group of the conductive traces 25A (C2) and 25A (C5) having small sectional areas.

When the thickness dimensions of the respective conductive traces 25A (C1) to 25A (C5) are increased, necessary electrical properties can be ensured while meeting the constraints of the outer dimensions of the guide wire 10.

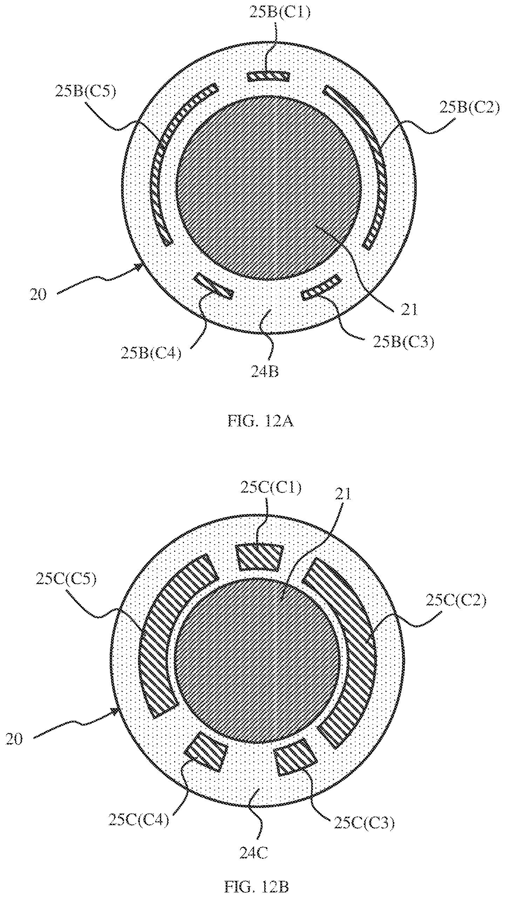

FIG. 12A and FIG. 12B are transverse-sectional views illustrating modification examples of the insulating layer that covers the guide wire core 20. There is a difference in the thickness dimensions of the conductive traces 25 between FIG. 12A and FIG. 12B. In FIG. 9 to FIG. 11, the examples of forming the plural insulating layers 24 and 26 over the longitudinal direction of the guide wire core 20 on the outer periphery side of the guide wire core 20 have been explained. Instead of this configuration, respective conductive traces 25B (C1) to 25B (C5) may be formed so as to be embedded in one insulating layer 24B, as illustrated in FIG. 12A. Respective conductive traces 25C (C1) to 25C (C5) may be formed so as to be embedded in one insulating layer 24C, as illustrated in FIG. 12B. That means, the inner insulating layers of the respective conductive traces 25B (C1) to 25B (C5) or the inner insulating layers of the 25C (C1) to 25C (C5) are the same as the outer insulating layers of the respective conductive traces. The surface of the insulating layer 24B or insulating layer 24C may be covered with yet another layer (not illustrated). In FIG. 12B, the conductive trace 25C can also be formed thicker than the conductive trace 25B illustrated in FIG. 12A, as described above.

The example in FIG. 12A includes two groups: a first group of the conductive traces 25B (C2) and 25B (C5) having large sectional areas; and a second group of the conductive traces 25B (C1), 25B (C3), and 25B (C4) having small sectional areas. The example in FIG. 12B includes two groups: a first group of the conductive traces 25C (C2) and 25C (C5) having large sectional areas; and a second group of the conductive traces 25C (C1), 25C (C3), and 25C (C4) having small sectional areas.

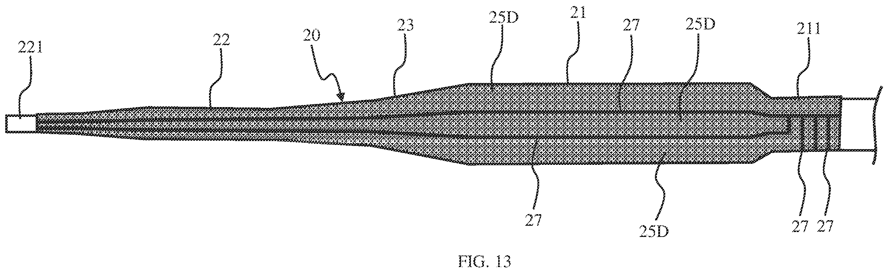

Some examples of the shape of the conductive traces 25 will be explained with reference to FIG. 13 to FIG. 15. FIG 13 illustrates an example that respective conductive traces 25D are formed in a straight shape in the longitudinal direction. The respective conductive traces 25D are formed in an almost straight shape on a region excluding the external connection portion 211 on the proximal end side and the sensor attachment portion 221 on the distal end side in the guide wire core 20.

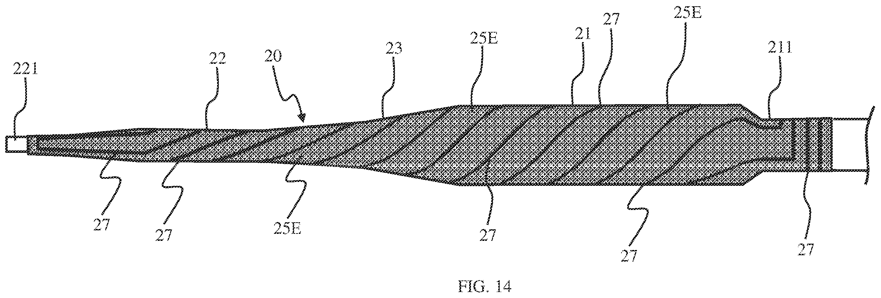

FIG. 14 illustrates an example in which respective conductive traces 25E are formed in a spiral shape on the lateral face (circumferential face) of the guide wire core 20. The conductive traces 25E are elongated by forming the respective conductive traces 25E in the spiral shape. However, in the example of FIG. 14, since locations where the shapes of the conductive traces 25E abruptly change can be reduced, an abrupt change in an impedance can be prevented, and generation of noises can be suppressed.

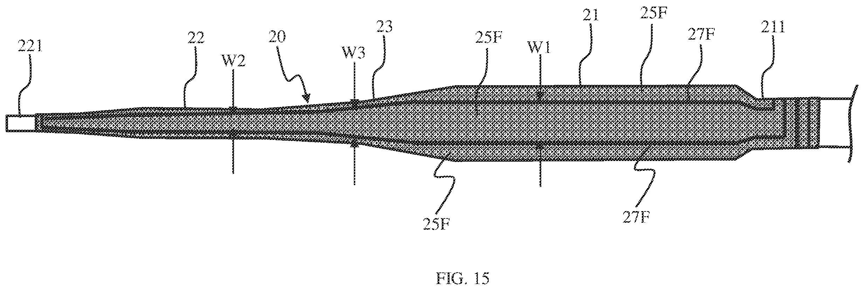

FIG. 15 illustrates another example in which width dimensions of respective conductive traces 25F vary in the longitudinal direction. The width dimensions of the respective conductive traces 25F vary in the longitudinal direction of the guide wire core 20 regardless of the variation in the diameter dimension of the guide wire core 20. That means, in the example of FIG. 15, a change rate in the diameter dimension of the guide wire core 20 along the longitudinal direction of the guide wire core 20 does not coincide with a change rate in the width dimensions of the conductive traces 25F along the longitudinal direction of the guide wire core 20. As for the respective conductive traces 25F, a width dimension W1 in the region formed on the large-diameter portion 21 is largest, a width dimension W2 in the region formed on the small-diameter portion 22 is smallest, and a width dimension W3 in the region formed on the tapered portion 23 gradually decreases toward the distal end side.

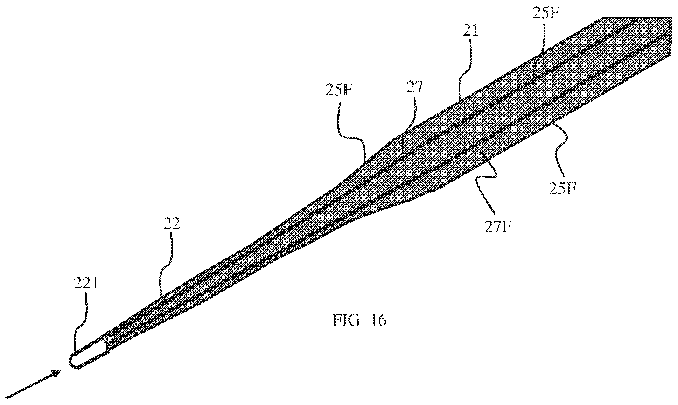

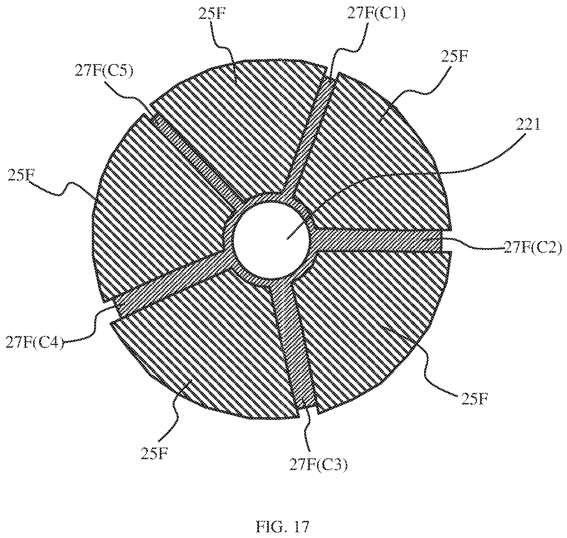

FIG. 16 is an enlarged view of the distal end in FIG. 15. FIG. 17 is a diagram viewed from a direction of the arrow in FIG. 16.

As illustrated in FIG. 17, width dimensions of gaps 27F between the respective conductive traces 25F are not necessarily equal, and may be different. That means, as illustrated in FIG. 17, at least one of the respective gaps 27F has a width dimension different from those of the other gaps 27F. In FIG. 17, identification numbers such as C1 and C2 are appended in parentheses accompanying the symbol 27F for the purpose of distinguishing the respective gaps 27F from each other.

Width dimensions of a gap 27F (C1) and a gap 27F (C5) are smaller than width dimensions of a gap 27F (C2), a gap 27F (C3), and a gap 27F (C4). Occurrence of a so-called crosstalk can be prevented by increasing the gaps 27F between the conductive traces 25F.

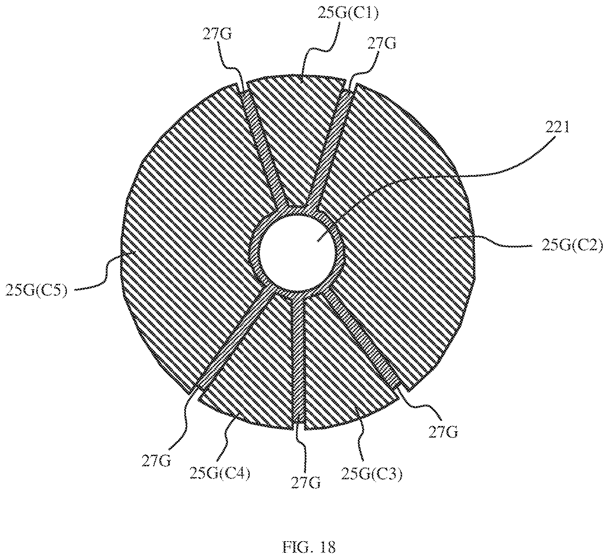

As a modification example, FIG. 18 is a diagram viewed from a direction of the arrow in FIG. 16. As illustrated in FIG. 18, width dimensions of gaps 27G can be uniformed, and width dimensions of conductive traces 25G can be varied. Although this configuration has been described above, the parasitic capacitance caused on the guide wire core 20 can be increased, and noises emitted from the guide wire core 20 can be decreased by increasing width dimensions of conductive traces 25G (C2) and 25G (C5) used as a power supply system (VCC, GND). The other conductive traces 25G (C1), 25G (C3), and 25G (C4) may be used as signal wires.

An example of a method for determining the sectional areas of the respective conductive traces 25 will be explained. For example, as in the conductive traces 25 used as the power supply system wirings (VCC, GND), the width dimensions of the conductive traces 25 having a restricted upper limit of a resistance value are set such that the resistance value is within a required resistance value range. From the remaining circumferential length, width dimensions are assigned to the other conductive traces used as the other wirings (e.g. signal wirings). For example, if a simulation or an experiment has a problem that a signal delay time is long in the conductive traces used as signal system wirings, increase in the thickness dimensions of the conductive traces is considered. In this way, in the first step, the width dimensions of the conductive traces are determined, and in the second step, the thickness dimensions of the conductive traces are determined. This makes it possible to achieve high functionality of the guide wire 10 while the increase in the diameter dimension of the guide wire 10 is suppressed as much as possible. However, the determination method described above is merely an example, and the dimensions can be determined according to another determination method. For example, in the first step, the thickness dimensions of the conductive traces may be determined based on electrical specifications required for the conductive traces, and in the second step, the width dimensions of the conductive traces may be determined.

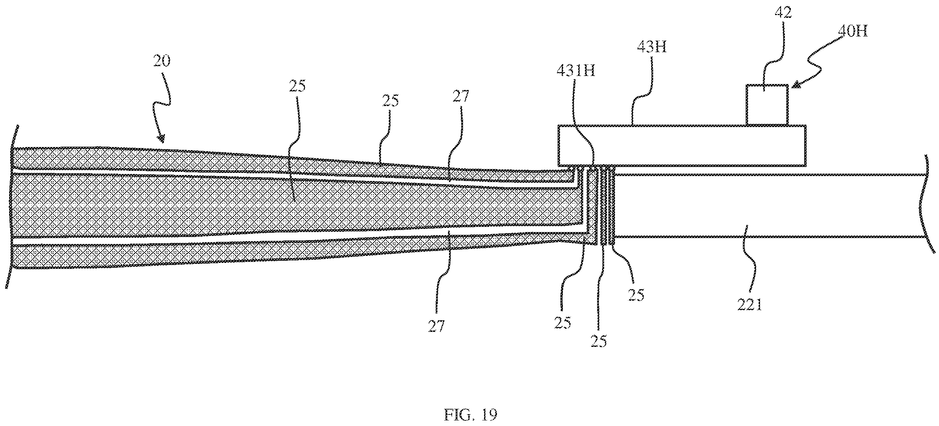

FIG. 19 illustrates an example that a sensor assembly 40H is attached to the guide wire core 20. FIG. 19 illustrates a case that, for example, electrical connection portions (e.g. pad) 431H of a wiring portion 43H formed as an interposer wiring board is provided on a surface opposite to a surface on which the sensor 42 is mounted. The electrical connection portions 431H of the wiring portion 43H are provided so as to face the guide wire core 20. In this case, as described above, the sensor assembly 40H (the sensor housing 41 is not illustrated in FIG. 19) only needs to be directly attached to the guide wire core 20 such that the respective electrical connection portions 431H of the wiring board 43H coincide with electrical connection portions 261 (not illustrated in FIG. 19) of the respective conductive traces 25.

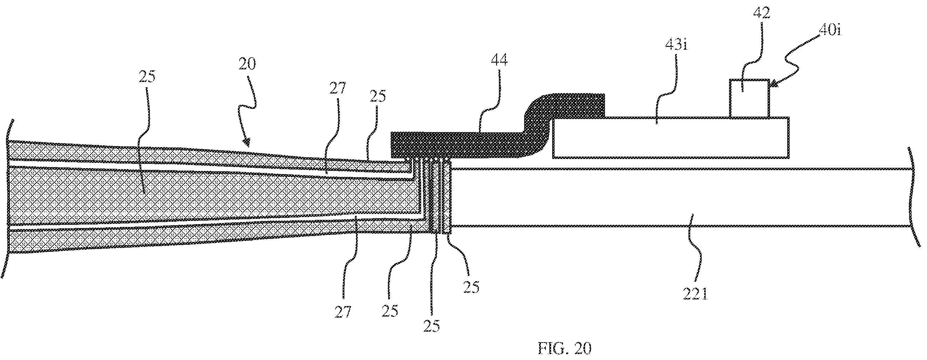

FIG. 20 illustrates another example that a sensor assembly 40i is attached to the guide wire core 20. FIG. 20 illustrates an example that the electrical connection portions (not illustrated) of a wiring portion 43i are provided on the same side as the face on which the sensor 42 is mounted. In this case, a flexible substrate 44 may be used to electrically connect the electrical connection portions of the wiring portion 43i to the electrical connection portions 261 (not illustrated in FIG. 20) of the respective conductive traces 25. A wire bonding technique using plural ultra-fine conductive wires instead of the flexible substrate 44 can also be employed. In FIG. 20, the sensor housing 41 is not illustrated.

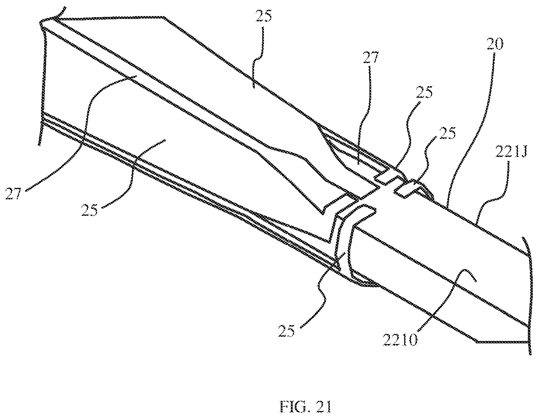

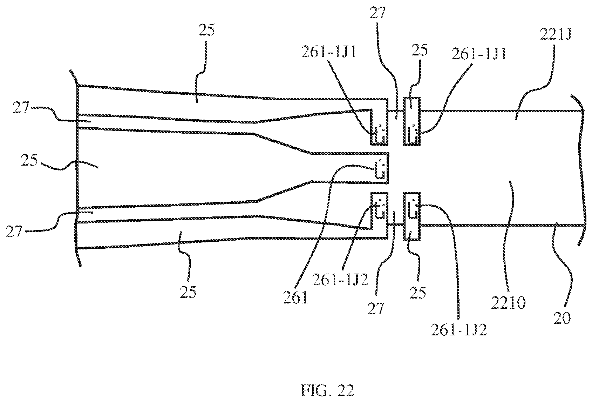

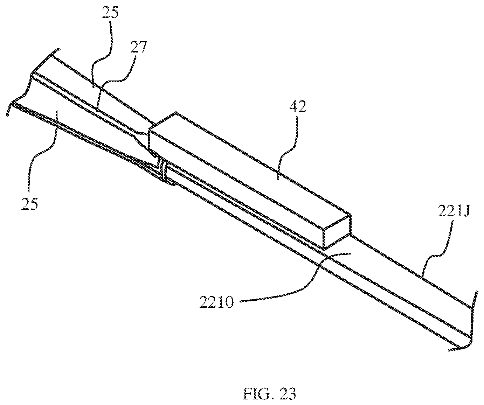

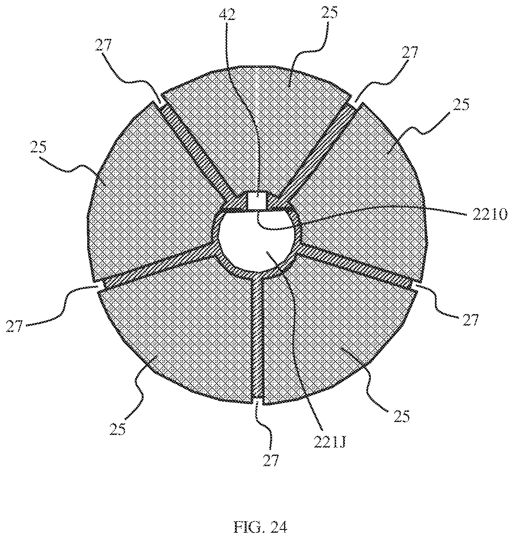

An example that a sensor attachment portion 221J of the guide wire core 20 has a flat portion 2210 will be explained with reference to FIG. 21 to FIG. 24. FIG. 21 is a perspective view of the sensor attachment portion 221J of the guide wire core 20. FIG. 22 is a plan view of the sensor attachment portion 221J. FIG. 23 is a perspective view illustrating a state that the sensor 42 is attached to the flat portion 2210 of the sensor attachment portion 221J. In FIG. 23, the sensor housing 41 is not illustrated. FIG. 24 is a front view of the sensor attachment portion 221F of the guide wire core 20 viewed from the distal end side. In FIG. 24, the sensor housing 41 is not illustrated.

The sensor attachment portion 221J having the flat portion 2210 is provided on the distal end side of the guide wire core 20. For example, a half part of the sensor attachment portion 221J is cut out along an axial center of the sensor attachment portion 221J to form the flat portion 2210. As a result, the sensor attachment portion 221J is formed into a semicylindrical shape. As illustrated in FIG. 24, the flat portion 2210 can also be formed by flattening a slightly upper side with respect to the axial center of the sensor attachment portion 221J.

As illustrated in FIG. 22, on the flat portion 2210, the distal end sides of the respective conductive traces 25 extend, and electrical connection portions 261-1J1 and 261-1J2 are formed. Herein, in the respective conductive traces 25, the two electrical connection portions 261-1J1 located on the upper side of FIG. 22 and the two electrical connection portions 261-1J2 located on the lower side of FIG. 22 are arranged in parallel on the flat portion 2210.

As illustrated in FIG. 23, an unillustrated terminal of the sensor 42 is electrically connected with the proximal-side electrical connection portions via the conductive traces 25.

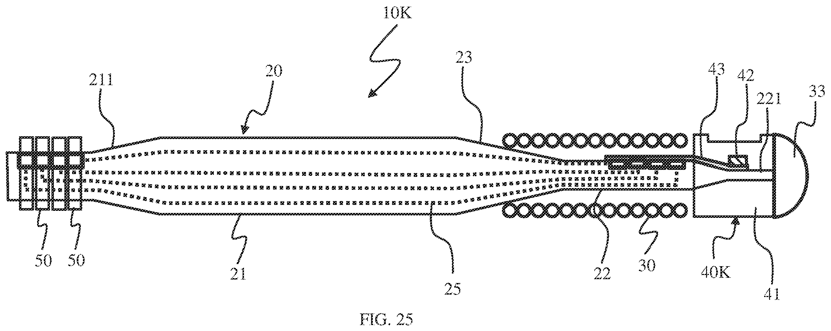

Some examples of mounting the sensor 42 to the guide wire 10 will be explained with reference to FIG. 25 to FIG. 29. In FIG. 25 to FIG. 29, the wiring portion 43 to which the sensor 42 is mounted is electrically connected to the respective conductive traces 25 on the sensor attachment portion 221 located on the distal end side of the guide wire core 20.

In the example of FIG. 25, a sensor assembly 40K is provided at a front end (distal end side) of a guide wire 10K. The front end tip 33 is provided on the distal end side of the sensor housing 41. In the example of FIG. 25, a coil body 30 is provided only on the proximal end side of the sensor assembly 40K.

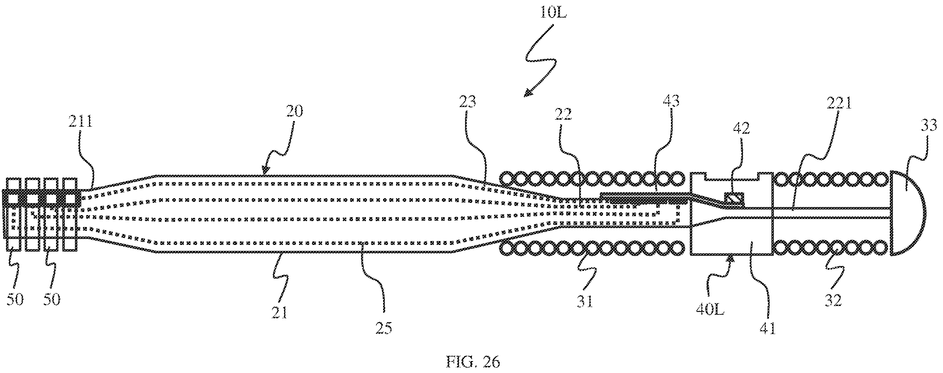

In a guide wire 10L illustrated in FIG. 26, as described above, the distal end of the guide wire core 20 is formed so as to penetrate a sensor assembly 40L, the coil body 31 is connected to the proximal end side of the sensor assembly 40L, and the coil body 32 is connected to the distal end side of the sensor assembly 40L.

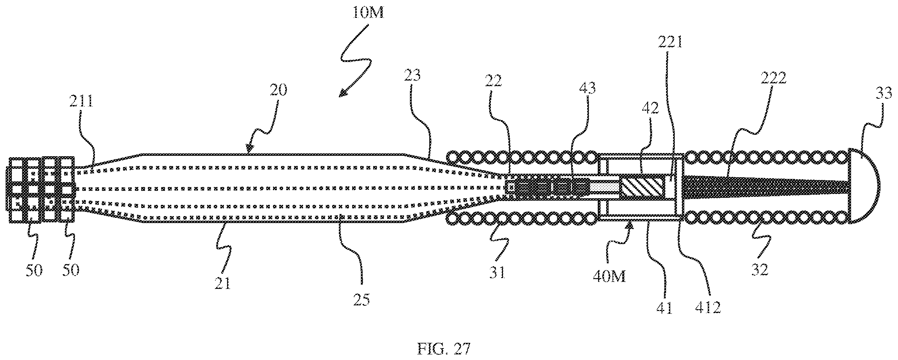

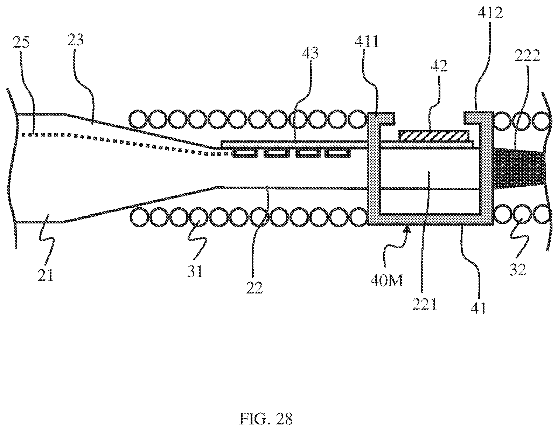

In a guide wire 10M illustrated in FIG. 27 and FIG. 28, the distal end of the guide wire core 20 is formed so as to extend to the distal end side of the sensor housing 41. Another core 222 (distal core 222) is connected to the distal end side of the sensor housing 41. That means, in the examples of FIG. 27 and FIG. 28, two cores, the distal core 222 and proximal core (a sensor attachment portion 221) are disposed with the wall portion 412 as a boundary on the distal end side of the sensor housing 41.

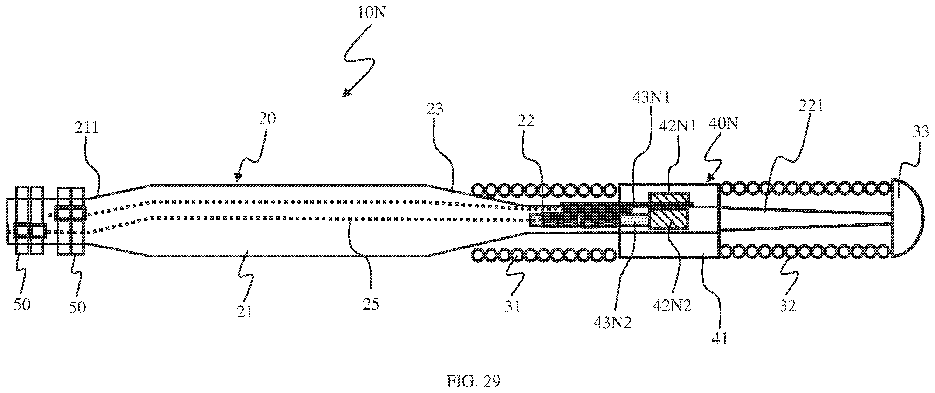

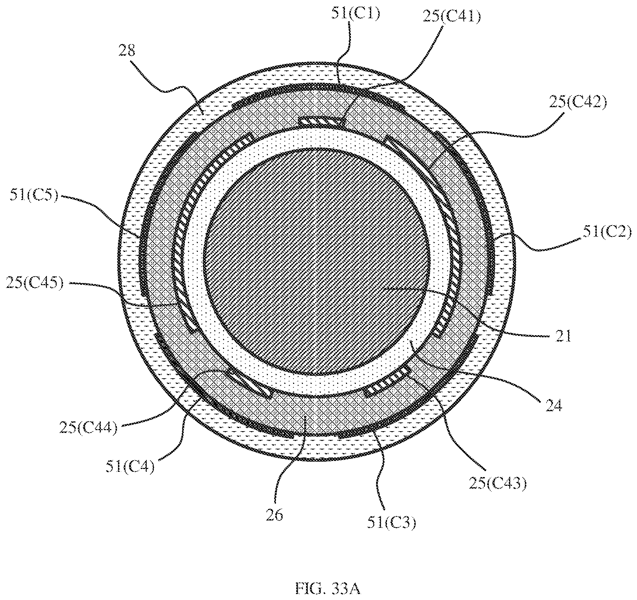

In a guide wire 10N of FIG. 29, plural conductive trace layers are formed on the guide wire core 20 in a build-up manner, and sensors 42N1 and 42N2 are connected to the respective conductive trace layers. Lamination of the plural conductive trace layers 25 and 51 (see FIG. 33A) onto the guide wire core 20 will be explained in detail below. Herein, the conductive layer on which the plural conductive traces are formed by a laser beam or the like is referred to as a conductive trace layer.

Respective wiring portions 43N1 and 43N2 are connected to the electrical connection portions (not illustrated) that are opened corresponding to the respective conductive trace layers. The sensors 42N1 and 42N2 are provided on the wiring portions 43N1 and 43N2 respectively. The respective sensors 42N1 and 42N2 are arranged spaced from each other in the lateral direction of the guide wire core 20. In FIG. 29, for example, one sensor 42N1 is provided on the upper side of the guide wire core 20 and the other sensor 42N2 is provided on the lateral side of the guide wire core 20. Also, three or more sensors can be provided by forming more conductive trace layers on the guide wire core 20.

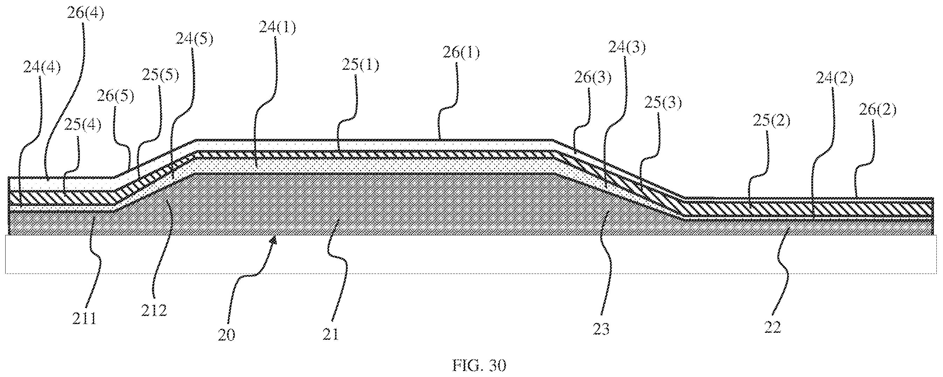

A variation in a longitudinal section of the guide wire will be explained with reference to FIG. 30 to FIG. 32. In the example of FIG. 30, the thickness dimensions of the conductive trace 25 (conductive trace layer), the first insulating layer 24, and the second insulating layer vary in the longitudinal direction of the guide wire core 20 (longitudinal direction of the guide wire 10). In FIG. 30 to FIG. 32, a structure corresponding to the large-diameter portion 21 of the guide wire core 20 is marked with symbol (1), a structure corresponding to the small-diameter portion 22 of the guide wire core 20 is marked with symbol (2), a structure corresponding to the tapered portion 23 is marked with symbol (3), a structure corresponding to the external connection portion 211 on the proximal end side of the large-diameter portion 21 is marked with symbol (4), and a structure corresponding to the tapered portion 212 that connects between the external connection portion 211 and the large-diameter portion 21 is marked with symbol (5).

In the example of FIG. 30, the conductive trace 25 is thicker in the regions corresponding to the external connection portion 211 and the small-diameter portion 22, and thinner in the region corresponding to the large-diameter portion 21. The tapered portions 23 and 212 on the distal and proximal end sides of the large-diameter portion 21 gradually vary in thickness. The large-diameter portion 21 has a large diameter dimension, and therefore has a large lateral area (area of the circumferential face) where the conductive trace layer can be formed. Since the width dimension of the conductive trace 25 can be increased on the large-diameter portion 21, it is possible to meet electrical constraints such as impedance even if the large-diameter portion 21 is thinned. In contrast, the small-diameter portion 22 and the external connection portion 211 have diameter dimensions smaller than of the large-diameter portion 21, and therefore have small lateral areas where the conductive trace 25 can be formed. Since the width of the conductive trace 25 cannot increased on the small-diameter portion 22 and the external connection portion 211, the conductive trace 25 is thickened. This makes it possible to meet the electrical constraints.

The thickness dimension of the first insulating layer 24 varies following the variation in the thickness dimension of the conductive trace layer so as to meet the constraints of the outside dimension of the guide wire 10. That means, the first insulating layer 24 is thinner in the region with the thicker conductive trace 25, and the first insulating layer 24 is thicker in the region with the thinner conductive trace 25.

Also, the thickness dimension of the second insulating layer 26 basically varies following the variation in the thickness dimension of the conductive trace layer so as to meet the constraints of the outside dimension of the guide wire 10. However, since the restrictions of the outside dimensions are loose on the butt side (proximal end side) of the guide wire 10, the second insulating layer 26 can be made thicker.

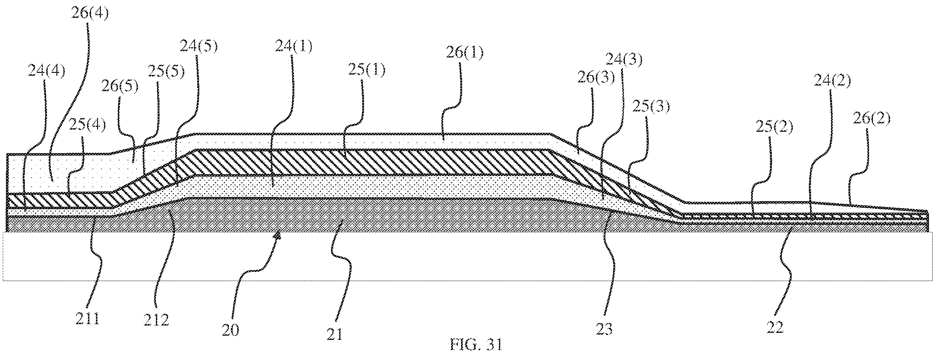

In the example of FIG. 31, the conductive trace 25 is thinner in the regions corresponding to the external connection portion 211 and the small-diameter portion 22, and thicker in the region corresponding to the large-diameter portion 21. The tapered portions 23 and 212 on the distal and proximal end sides of the large-diameter portion 21 gradually vary in thickness. The thickness dimension of the first insulating layer 24 generally follows the variation in the thickness dimension of the conductive trace 25.

Since the constraints of the external dimension on the external connection portion 211 are looser than those on the small-diameter portion 22, the thickness dimensions of the conductive trace 25 and the second insulating layer 26 on the external connection portion 211 can be made larger than those on the small-diameter portion 22. In this example, since the conductive trace 25 can be made thicker on the large-diameter portion 21 that accounts for the largest proportion of the total length of the guide wire 10, the impedance can be decreased to improve the noise resistance.

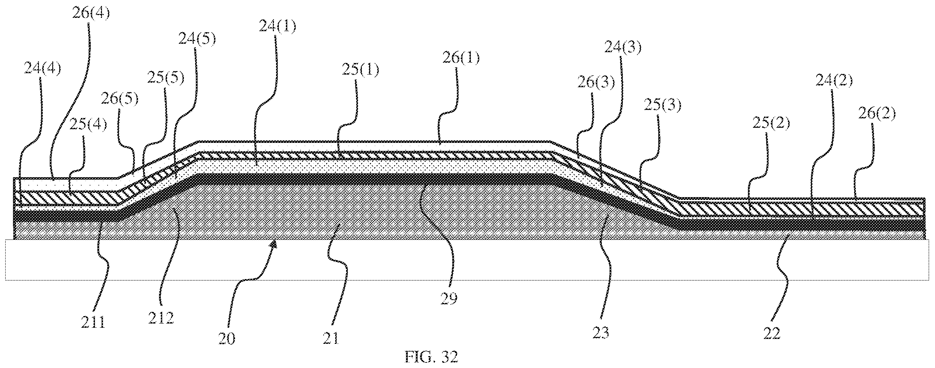

In the example of FIG. 32, a metal layer 29 having high conductivity (high-conductivity metal layer) is formed between the first insulating layer 24 and the surface of the guide wire core 20. In other words, in the example of FIG. 32, first, the high-conductivity metal layer 29 is formed on the surface of the guide wire core 20, the first insulating layer 24 is formed on the surface of the high-conductivity metal layer 29, the conductive trace layer is formed on the surface of the first insulating layer 24, and the second insulating layer 26 is formed so as to cover the first insulating layer 24 and the conductive trace layer.

For example, when the guide wire core 20 is made of a conductive material such as stainless steel, the guide wire core 20 can be used alone as a ground electrode. For the purpose of further increasing the conductivity, the high-conductivity metal layer 29 may be formed on the surface of the guide wire core 20. The high-conductivity metal layer 29 is not limited to metals such as copper, gold, and silver. The high-conductivity metal layer 29 may be made of a conductive polymer. Examples of the conductive polymer includes, but are not limited to, polypyrrole, polythiophene, polyacetylene, and polyaniline. The high-conductivity metal layer 29 is formed on the surface of the guide wire core 20, so that a return path for using the guide wire core 20 as a ground layer (GND) can be endured. The guide wire core 20 alone can be used as a ground electrode.

The variation in the thickness dimensions of the first insulating layer 24, the conductive trace layer, and the second insulating layer 26 in FIG. 32 is the same as in the example of FIG. 30. Instead, the variation in the thickness dimensions of the layers 24, 25, and 26 may be made the same as in the example of FIG. 31.

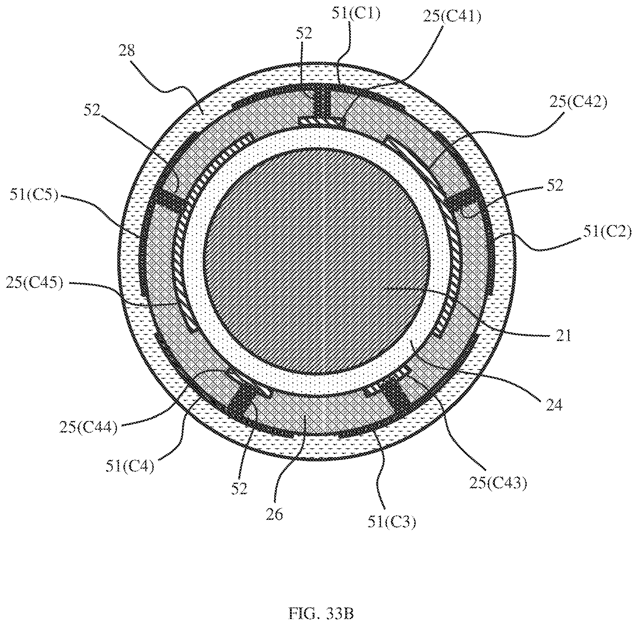

FIG. 33A and FIG. 33B are transverse-sectional views of the guide wire in which the plural conductive layers 25 and 51 are formed in a build-up manner. For the purpose of facilitating distinction, the conductive traces 25 are marked with identification numbers (C41) to (C45), and the conductive layers 51 are marked with identification numbers (C1) to (C5). For example, the conductive traces 25 are connected to the first sensor 42N1 illustrated in FIG. 29, and the conductive layers 51 are connected to the second sensor 42N2 illustrated in FIG. 29.

In the example of FIG. 33A, the first insulating layer 24, the layers of the conductive traces 25 (that may be also referred to as the first conductive trace layers), the second insulating layer 26, the second conductive layers 51 (that may be also referred to as the second conductive trace layers) and the third insulating layer 28, are formed in this order on the surface of the guide wire core 20. Similarly to the respective conductive traces 25, the plural conductive layers 51 are arranged spaced from each other in the lateral direction of the guide wire core 20. In the example of FIG. 33A, the layers of the conductive traces 25 and the conductive layers 51 are not electrically connected. The layers of the conductive traces 25 and the conductive layers 51 constitute mutually-independent wirings. That means, the electrical wirings of the first sensor 42N1 (electrical wirings formed as the conductive traces 25) and the electrical wirings of the second sensor 42N2 (electrical wirings formed on the conductive layers 51) are independent of each other.

Also in the example of FIG. 33 B, the first insulating layer 24, the layers of the conductive traces 25, the second insulating layer 26, the second conductive layers 51, and the third insulating layer 28 are formed in this order on the surface of the guide wire core 20. Similarly to the respective conductive traces 25, the plural conductive layers 51 are arranged spaced from each other in the lateral direction of the guide wire core 20. Unlike the example of FIG. 33A, in the example of FIG. 33B, at least a part of the conductive trace 25 and a part of the corresponding conductive layer 51 are electrically connected to each other e.g. via a via hole 52. That means, in the example of FIG. 33B, a part of the electrical wiring of the first sensor 42N1 and a part of the electrical wiring of the second sensor 42N2 are electrically connected to each other via the via hole 52. The thickness dimension of the conductive trace 25 and the thickness dimension of the conductive layer 51 may be the same or different from each other. Each ratio between a width dimension of one conductive trace 25 and a width dimension of a corresponding conductive layer 51 may be set to the same ratio among all pairs, or set to different ratios from each other. In other words, their width dimensions can be set for a pair of a small-width conductive trace 25 and a small-width conductive layer, a pair of a large-width conductive trace 25 and a large-width conductive layer, a pair of a small-width conductive trace 25 and a large-width conductive layer, a pair of a large-width conductive trace 25 and a small-width conductive layer, or the like.

In FIG 33A, for example, the conductive trace 25 (C41) is used as a wiring (CLK) for flowing clocks, and the conductive traces 25 (C43) and 25 (C44) are used as wirings (MOSI, MISO) for flowing signals. MOSI means master output/slave input. MISO means master input/slave output. The other conductive traces 25 (C42) and 25 (C45) are used as power supply system wirings (VCC, GND).

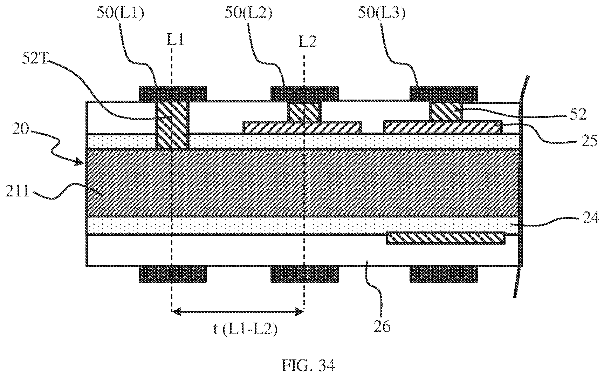

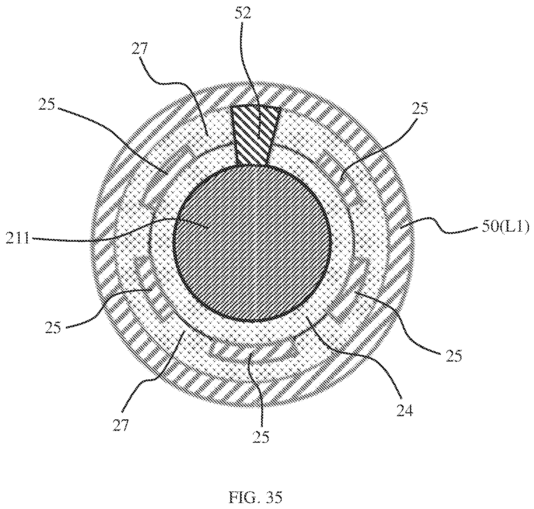

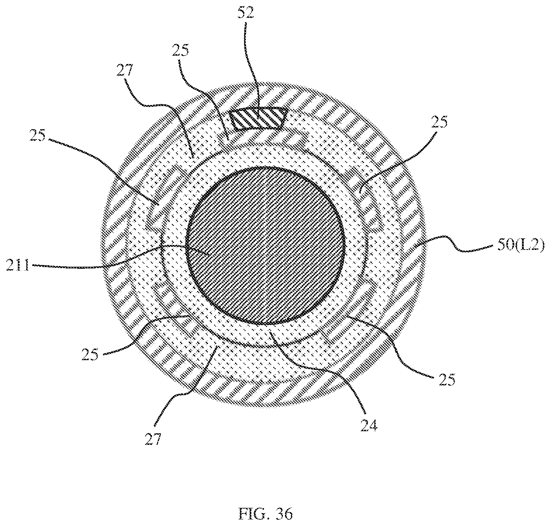

FIG. 34 is a longitudinal sectional view of the guide wire illustrating a relationship between the conductive traces 25 and the ring electrodes 50. FIG. 35 is a sectional view taken along line L1 in FIG. 34. FIG. 36 is a sectional view taken along line L2 in FIG. 34. FIG. 34 illustrates some ring electrodes 50 (L1), 50 (L2), and 50 (L3) of the plural ring electrodes 50 disposed on the proximal end side of the guide wire core 20. The identification numbers (L1), (L2), and (L3) are appended for the purpose of distinguishing the respective ring electrodes 50. The ring electrodes 50 and the via holes 52 may be formed as separate members, or integrated, as illustrated in FIG. 34 to FIG. 36 and the like.

As illustrated in FIG. 35, a ring electrode 50 (L1) located on the most proximal end side is used as a ground electrode and is electrically connected to the guide wire core 20 via the via hole 52. As illustrated in FIG. 36, a ring electrode 50 (L2) adjacent to the distal end side of the ring electrode 50 (L1) is electrically connected to the corresponding conductive trace 25 via the via hole 52. Since the ring electrode 50 (L1) is electrically connected to the guide wire core 20 via a via hole 52T, and the other ring electrodes 50 (L2) and 50 (L3) are connected to the conductive trace 25 via the via hole 52, and therefore a distance t (L1-L2) between the ring electrode 50 (L1) and the adjacent ring electrode 50 (L2) tends to be wider than the distance between the other ring electrodes 50.

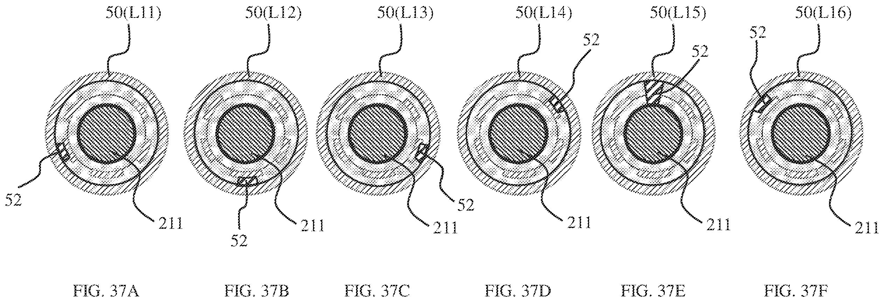

FIG. 37 is a sectional view illustrating the relationship between the conductive traces 25 and the ring electrodes 50, in an order from the distal end of the guide wire. In this example, among the set of the ring electrodes 50, a ring electrode 50 (L15) of the second order from the distal end side is connected to the guide wire core 20 via the via hole 52 and is used as a ground electrode. The other ring electrodes 50 (L11), 50 (L12), 50 (L13), 50 (L14), and 50 (L16) are connected to the corresponding conductive traces 25 via the via holes 52.

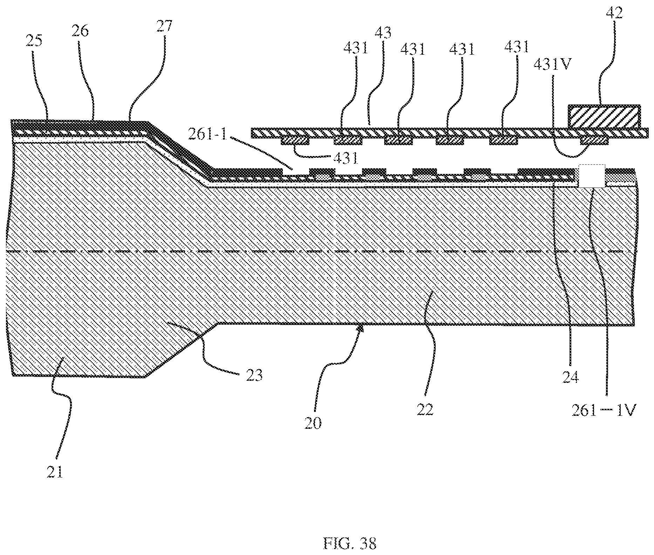

FIG. 38 is a longitudinal sectional view illustrating an example that the guide wire core 20 is used as an electrical wiring (e.g. ground wiring). In FIG. 38, the insulating layers 24 and 26 and the conductive traces 25 are illustrated only on the upper half of the guide wire core 20. The guide wire core 20 is made of the aforementioned conductive material, and is used as a ground wiring. A pad 431V for ground connection is formed on a pad-forming face (face opposed to the guide wire core 20) of the wiring portion 43. The pad 431V for ground connection is attached to an electrical connection portion 261-1V provided on the small-diameter portion 22 using a conductive adhesive, a solder, or the like. The electrical connection portion 261-1V is formed so as to penetrate the first insulating layer 24 and the second insulating layer 26. Thereby, the sensor 42 is electrically connected to the guide wire core 20 as a ground wiring.

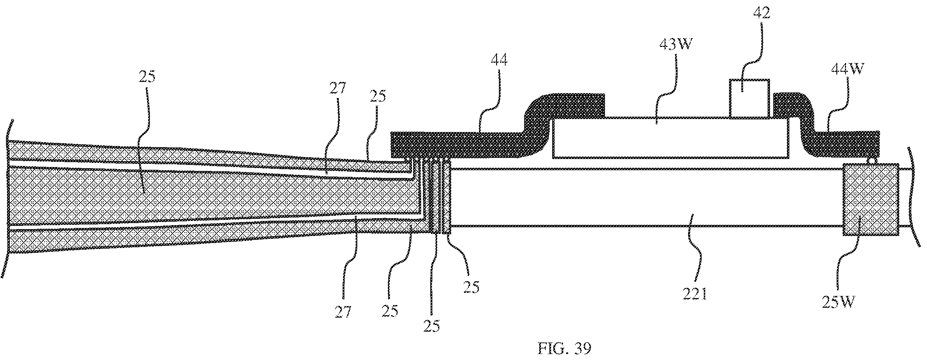

FIG. 39 is a longitudinal sectional view illustrating another example that the guide wire core 20 is used as a ground electrode. The guide wire core 20 is made of the aforementioned conductive material, and is used as a ground wiring. In this example, a wiring portion 43W is electrically connected to a conductive trace pad 25W formed on the small-diameter portion 22 of the guide wire core 20 via another flexible substrate 44W The conductive trace pad 25W is a pad composed of a conductive trace formed separately from the respective conductive traces 25. The sensor 42 is electrically connected to the guide wire core 20 as the ground wiring via another flexible substrate 44W and the conductive trace pad 25W.

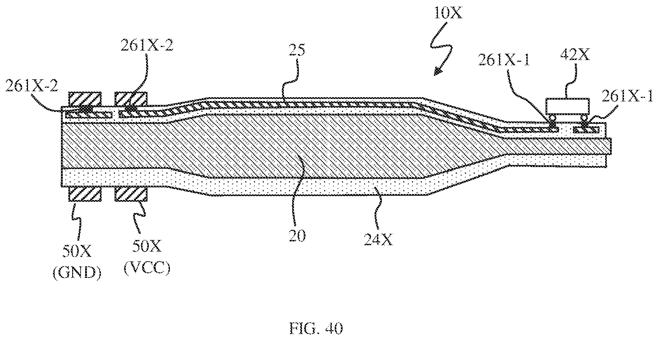

FIG. 40 to FIG. 43 are schematic diagrams illustrating an example of electrical connection of the guide wire. FIG. 40 to FIG. 43 illustrate a power supply system wiring, and illustrate no signal wiring.

FIG. 40 is a schematic diagram illustrating an example of electrical connection of a guide wire 10X. On the distal end side of the guide wire core 20, each terminal of a sensor 42X is electrically connected to the conductive traces 25 via electrical connection portions 261X-1. On the proximal end side of the guide wire core 20, the conductive traces 25 and ring electrodes 50X are electrically connected to each other via electrical connection portions 261X-2. For example, a ring electrode 50X (GND) on the most proximal end side is a ground electrode that is connected to the conductive trace 25 used as the ground wiring. A ring electrode 50X (VCC) adjacent to the distal end side of the ring electrode 50X (GND) used as the ground electrode is a positive electrode connected to the conductive trace 25 used as a positive power supply wire. The guide wire core 20 and the conductive traces 25 are covered by an insulating layer 24X. The insulating layer 24X may be multi-layered.

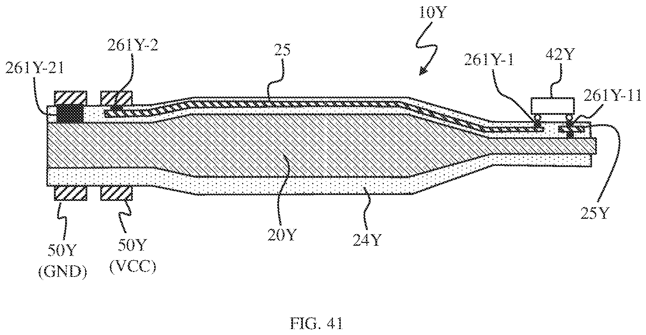

FIG. 41 is a schematic diagram illustrating another example of electrical connection of a guide wire 10Y In this example, a guide wire core 20Y is made of a conductive material. The guide wire core 20Y is used as a ground wiring. On the distal end side of the guide wire core 20Y, a ground terminal of a sensor 42Y is electrically connected to the guide wire core 20Y via a conductive trace 25Y and an electrical connection portion 261Y-11. The conductive trace 25Y is formed for connection to the ground wiring. The conductive trace 25Y is formed separately from the conductive trace provided as the VCC wiring. On the distal end side of the guide wire core 20Y, a positive terminal of the sensor 42Y is electrically connected to a conductive trace 25 via an electrical connection portion 261Y-1. On the proximal end side of the guide wire core 20Y, a ring electrode 50Y (GND) used as a ground electrode is electrically connected to the guide wire core 20Y via an electrical connection portion 261Y-21. The electrical connection portion 261Y-21 is formed such that its sectional area is wide as much as possible to decrease an impedance. A ring electrode 50Y (VCC) used as a positive electrode is electrically connected to the conductive trace 25 via an electrical connection portion 261Y-2. The guide wire core 20Y and the conductive traces 25 and 25Y are covered by an insulating layer 24Y. The insulating layer 24Y may be multi-layered.

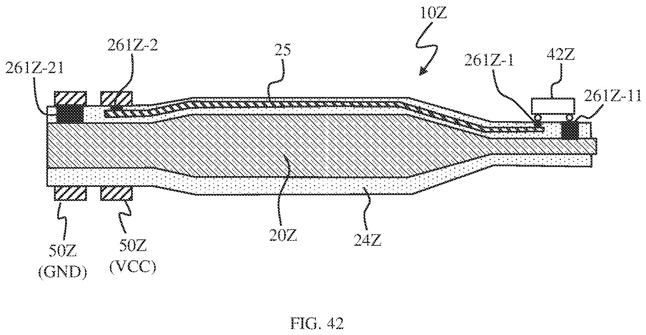

FIG. 42 is a schematic diagram illustrating yet another example of electrical connection of a guide wire 10Z. In this example, a guide wire core 20Z is made of a conductive material. The guide wire core 20Z is used as a ground wiring. On the distal end side of the guide wire core 20Z, a ground terminal of a sensor 42Z is electrically connected to the guide wire core 20Z via an electrical connection portion 261Z-11. On the distal end side of the guide wire core 20Z, a positive terminal of the sensor 42Z is electrically connected to the conductive trace 25 via an electrical connection portion 261Z-1. On the proximal end side of the guide wire core 20Z, a ring electrode 50Z (GND) used as a ground electrode is electrically connected to the guide wire core 20Z via an electrical connection portion 261Z-21. The electrical connection portions 261Z-11 and 261Z-21 are formed such that their sectional areas are wide as much as possible to decrease an impedance. A ring electrode 50Z (VCC) used as a positive electrode is electrically connected to the conductive trace 25 via an electrical connection portion 261Z-2. The guide wire core 20Z and the conductive trace 25 are covered by an insulating layer 24Z. The insulating layer 24Z may be multi-layered.

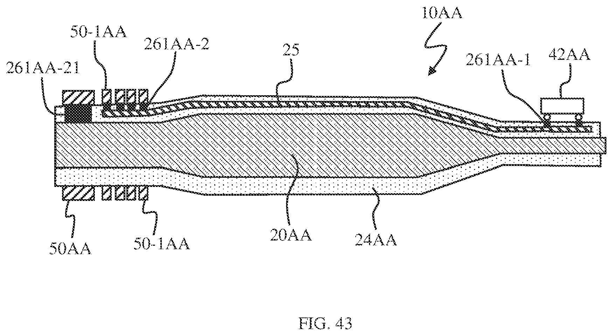

FIG. 43 is a schematic diagram illustrating another example of an electrical connection of a guide wire 10AA. In this example, a guide wire core 20AA is made of a conductive material. On the distal end side of the guide wire core 20AA, a terminal of a sensor 42AA is electrically connected to the conductive trace 25 via an electrical connection portion 261AA-1. On the proximal end side of the guide wire core 20AA, the respective conductive traces 25 are connected to respective corresponding ring-shaped terminals 50-1AA via electrical connection portions 261AA-2. That means, in this example, the sensor 42AA is not directly connected to the guide wire core 20AA. A ring electrode 50AA may be electrically connected to the guide wire core 20AA via an electrical connection portion 261AA-21. The guide wire core 20AA and the conductive trace 25 are covered by an insulating layer 24AA. The insulating layer 24AA may be multi-layered.

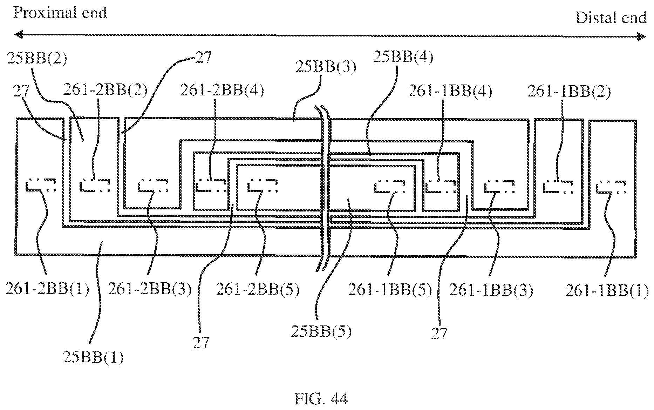

FIG. 44 illustrates an example that the plural conductive traces are deployed into a plane. In FIG. 44, the guide wire core is not illustrated, and the proximal and distal end directions are illustrated. Respective conductive traces 25BB (1) to 25BB (5) are formed in a nest structure in an order from the outside in the longitudinal direction. An electrical connection portion 261-1BB (1) and an electrical connection portion 261-2BB (1) are formed on both end sides of the conductive trace 25BB (1) located on the outermost side in the longitudinal direction. An electrical connection portion 261-1BB (2) and an electrical connection portion 261-2BB (2) are formed on both ends of the conductive trace 25BB (2) adjacent to the outermost conductive trace 25BB (1) via the gap 27. Likewise, an electrical connection portion 261-1BB (3) and an electrical connection portion 261-2BB (3) are formed on both end sides of the conductive trace 25BB (3) located on the outermost side in the longitudinal direction. An electrical connection portion 261-1BB (4) and an electrical connection portion 261-2BB (4) are formed on both end sides of the conductive trace 25BB (4). An electrical connection portion 261-1BB (5) and an electrical connection portion 261-2BB (5) are formed on both end sides of the conductive trace 25BB (5) located on the innermost side in the longitudinal direction.

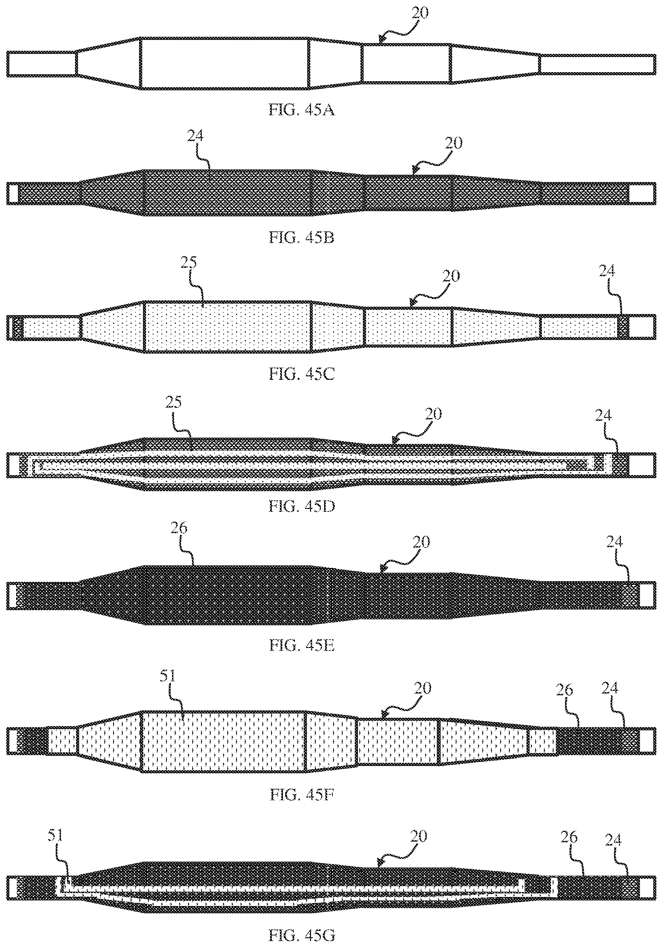

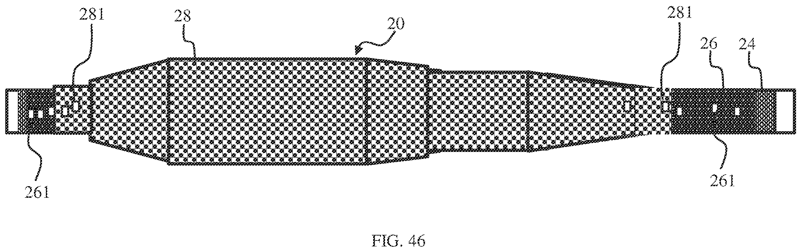

An approach for building the plural conductive trace layers into separate insulating layers to incorporate the plural conductive traces 25 into the guide wire 10 will be explained with reference to FIG. 45 and FIG. 46. By this method, plural lead wires can be incorporated into a relatively narrow space of a guide wire or a catheter-type device. This method is particularly effective when plural diagnostic sensors are intended to be incorporated into one guide wire, and requires an innovative way for forming signal wires in a narrow space without affecting primary mechanical performances of a device.

It is difficult to incorporate a conductive element into a typical 0.014 inch (0.356 mm)-diameter guide wire core 20 without affecting mechanical properties such as followability and torque responsibility. Use of a layering manufacture method as described in patent application No. US20190821 makes it possible to form a conductive element directly on a core for the purpose of maintaining basic mechanical performances of a guide wire device. However, it is very difficult to incorporate more conductive elements, e.g. four or more conductive elements (conductive trace layers) into the typical guide wire core 20 having a diameter of 0.014 inches (0.356 mm) or smaller. If two or more types of sensors, or a sensor requiring four or more independent communication channels should be incorporated into one device, it is beneficial to have four or more different signaling elements in one device in some cases. To achieve this configuration, a layering approach as described below is effective. The present disclosure is applied to not only to the 0.014-inch guide wire core 20 but also to another guide wire core 20 having a typical diameter dimension.

A typical guide wire core is illustrated in FIG. 45A. The guide wire core 20 has plural diameters and tapers, and the distal diameter of the guide wire core 20 is typically smaller than the diameters of other parts of the guide wire core 20. A material for the core is typically stainless steel (SS), Nitinol, or a combination thereof.

As illustrated in FIG. 45B, the first insulating layer 24 is formed on the metal guide wire core 20. The first insulating layer 24 can be formed by various methods such as dip coating, spray coating, PVD (Physical Vapor Deposition), CVD (Chemical Vapor Deposition), printing, and melt reflow. Examples of the polymer as the material for the first insulating layer 24 include polyimide, PET, nylon, Pebax, and the like.

As illustrated in FIG. 45C, subsequently, the first conductive trace layer as the first conductive layer is formed on the first insulating layer 24. As one method, first, a seed conductive layer such as palladium and silver is applied on the first insulating layer 24, on which subsequently a layer of a highly conductive metal such as copper and gold is used using electroless plating or electroplating.

As illustrated in FIG. 45D, subsequently, the first conductive trace layer is selectively etched to form individual conductive traces 25 that are each electrically insulated. As one method for achieving this configuration, a conductor is cut into individual traces, using a laser.

As illustrated in FIG. 45E, subsequently, the second insulating layer 26 is applied on the electrically insulated conductors. Examples of the insulating polymer include polyimide, PET, nylon, Pebax, and the like.

As illustrated in FIG. 45F, the second conductive trace layer as the second conductive layer is formed on the previously-formed second insulating layer 26. In a scenario, this metal layer can serve as a shielding layer. In this case, the third insulating layer can be overlaid on the second conductive trace layer, which can be further processed by forming the third conductive layer on the third insulating layer. In another scenario, when the shielding layer is not required, the second conductive layer may be further processed into a circuit pattern having individual electrically insulated conductors, using the aforementioned technique.

As illustrated in FIG. 45G, the second conductive trace layer is processed to form the second conductive traces 51. The circuit pattern of the second conductive traces 51 is formed such that pad patterns are radially aligned or radially displaced, in some cases. FIG. 45G illustrates a radial in-line pattern.

As illustrated in FIG. 46, subsequently, the third insulating layer 28 is formed on the formed second impure conductive element.

Next, opening portions are formed on the second insulating layer 26 and the third insulating layer 28 by etching, laser ablation, or the like, to form vias for accessing the corresponding conductive traces directly beneath the insulating layer. These vias constitute connection pads for connecting or combining the formed conductive traces to the outside of the guide wire 10. Thereby, the conductive traces are connected to e.g. one or more sensors located on the distal end of the guide wire, the connection terminal located on the appropriate proximal end, or the like.

In another embodiment, the vias 52 may be formed such that specific conductive traces on the first conductive layer are connected to specific conductive traces or the ring electrodes 50 on the second conductive layer. This configuration can be made for the purpose of decreasing the impedance or connecting specific sensor terminals from two different sensors to a common input conductor, a common same signal output conductor, ground plane, or the like. The sensors connected to the common input may be adjusted to use a single input signal transmitted by a common conductor, or one or more different signals transmitted within the conductor. For example, plural input signals separated in a time domain and/or in a frequency domain for enabling identification of information directed to the respective sensors and preventing loss of the information may transmit in a single conductor. Similarly, the output signals from the plural sensors are connected to a single output conductor, and the signals may be combined by a method for irreversibly combining the outputs of the sensors, or a method for allowing the output signals to be separated such that information from the outputs of the respective sensors are maintained and not lost. For example, the output signals can be separated in a time domain and/or a frequency domain such that information from the respective sensors are not lost and can be identified.

Since radial spaces are often limited, the sensors can be directly attached to the formed connection pads by using the wiring portion 43 such as a flex wiring board as a combining medium between the sensors and the connection pads on the distal end. To combine the plural sensors in a common space, a long flex circuit capable of connecting with the plural sensors or plural flex circuits are used, and the flex circuit (s) is oriented in the radial direction so as to fit the spaces, so that a common guide wire main body can be formed. Also, a metal housing for sealing the sensors, the flex wiring board, and a portion of the guide wire may be used.

In a further embodiment, the plural sensors may be attached to a single conductive element layer via a single flex connector, and input signals to be transmitted to the sensors may be kept separable, identifiable, and usable using one or more sensors by a multiplexing technique such as variation in frequency and/or time domain. Similarly, plural output signals from one or more sensors transmit in a single conductor, and can be kept separable, identifiable, and usable by the multiplexing technique such as variation in frequency and/or time domain.

The guide wire core 20 of the guide wire 10 can be terminated in the vicinity the distal end of the sensor housing 41 within the sensor housing 41, and thereby additional spaces for accommodating the sensor 42 can be made within the sensor housing 41 on the distal end of the guide wire core 20. If the size of the sensor permits, a suitably-sized continuous core may thoroughly penetrate the sensor housing 41. The sensor housing 41 may include a concave portion for accommodating the sensor 42, and an opening portion for enabling communication between the sensor 42 and an outside environment of the sensor housing 41. The sensor housing 41 may further include a distal core wire to which an attractive coil can be attached. Alternatively, the sensor housing 41 may be a hollow tube regardless of presence/absence of an opening portion to the outside, and a second distal core wire may be attached to the distal end of the sensor housing 41, allowing attachment of an atraumatic distal coil to the end portion of the guide wire e.g. via attachment of the coil to the distal end of the distal core and the distal end of the sensor housing 41 and/or via attachment of the coil to the proximal end of the distal core wire.

An approach with the aforementioned layered structure also makes it possible to form third and fourth conductive layers having individual conductors, depending on an application and dimensions. We were able to achieve formation of an electrically insulated two-layered conductive trace with a thickness of 7.5 µm or a diameter of 15 µm.

## Claims

1. A guide wire (10) comprising:
a guide wire core (20) comprising:
a large-diameter portion (21),
a small-diameter portion (22) located distal to the large-diameter portion (21) and having an outer dimension that is smaller than an outer dimension of the large-diameter portion (21), and a tapered portion (23) located between the large-diameter portion (21) and the small-diameter portion (22),
a first insulating layer (24) provided on a surface of the guide wire core (20), and
plural conductive traces (25) provided spaced from each other in a circumferential direction of the guide wire core (20) on a surface of the first insulating layer (24), **characterised in that**
a width dimension and/or a thickness dimension of at least one of the plural conductive traces (25) change on the tapered portion (23), and
in the large-diameter portion (21) and the small-diameter portion (22), a longitudinal extension of at least one of the plural conductive traces (25) has a sectional area different from those of a longitudinal extensions of the other conductive traces in a transverse-sectional view of the guide wire core (20).

2. The guide wire (10) according to claim 1, wherein
at least one of the plural conductive traces (25) has a thickness dimension different from those of the other conductive traces in the transverse-sectional view of the guide wire core (20) at different positions in a length direction.

3. The guide wire (10) according to claim 1, wherein
at least one of the plural conductive traces (25) has a width dimension different from those of the other conductive traces in the transverse-sectional view of the guide wire core (20) at different positions in a length direction.

4. The guide wire (10) according to claim 1, wherein
among gaps (27) between the plural conductive traces (25), gaps on the small-outer diameter portion of the guide wire core (20) have width dimensions larger than those of gaps on the large-outer diameter portion of the guide wire core (20) in the transverse-sectional view of the guide wire core (20).

5. The guide wire (10) according to claim 1, wherein
among gaps between the plural conductive traces (25), gaps on the small-outer diameter portion of the guide wire core (20) have width dimensions smaller than those of gaps on the large-outer diameter portion of the guide wire core (20) in the transverse-sectional view of the guide wire core (20).

6. The guide wire (10) according to claim 1, wherein
at least one of the plural conductive traces has a changed shape in the transverse-sectional view of the guide wire core (20) at different positions in a length direction.

7. The guide wire (10) according to claim 6, wherein
a width dimension of at least one of the plural conductive traces (25) changes on the small-diameter portion (22) in the transverse-sectional view of the guide wire core (20) at different positions in the length direction.

8. The guide wire (10) according to claim 6, wherein
a thickness dimension of at least one of the plural conductive traces (25) changes on the small-diameter portion (22) in the transverse-sectional view of the guide wire core (20).

9. The guide wire (10) according to claim 1, wherein
each of the plural conductive traces (25) has each electrical connection portion (261-1, 261-2), and the plural electrical connection portions (261-1, 261-2) are arranged on one straight line of the guide wire core (20).

10. The guide wire (10) according to claim 1, comprising
a second insulating layer (26) that covers the first insulating layer (24) and the plural conductive traces (25), wherein
the first insulating layer (24) is made of a material having a lower dielectric constant than of the second insulating layer (26).

11. The guide wire (10) according to claim 1, comprising
a second insulating layer (26) that covers the first insulating layer (24) and the plural conductive traces (25), wherein
the second insulating layer (26) is made of a material having a higher moisture resistance than of the first insulating layer (24).

12. The guide wire (10) according to claim 1, comprising
a second insulating layer (26) that covers the first insulating layer (24) and the plural conductive traces (25); and
a conductive layer provided on a surface of the second insulating layer (26), wherein
the conductive layer is electrically connected to at least one of the plural conductive traces.

13. The guide wire (10) according to claim 1, comprising
a second insulating layer (26) that covers the first insulating layer (24) and the plural conductive traces (25); and
a conductive layer provided on a surface of the second insulating layer (26), wherein a part of the conductive layer is electrically connected to the guide wire core (20).

## Patentansprüche

1. Ein Führungsdraht (10), umfassend:
einen Führungsdrahtkern (20), umfassend:
einen großdurchmessrigen Abschnitt (21),
einen kleindurchmessrigen Abschnitt (22), der distal zum großdurchmessrigen Abschnitt (21) angeordnet ist und eine äußere Abmessung aufweist, die kleiner ist als eine äußere Abmessung des großdurchmessrigen Abschnitts (21), sowie einen konischen Abschnitt (23), der zwischen dem großdurchmessrigen Abschnitt (21) und dem kleindurchmessrigen Abschnitt (22) angeordnet ist,
eine erste Isolierschicht (24), die auf einer Oberfläche des Führungsdrahtkerns (20) vorgesehen ist, und
mehrere leitfähige Leiterbahnen (25), die in Umfangsrichtung des Führungsdrahtkerns (20) auf einer Oberfläche der ersten Isolierschicht (24) voneinander beabstandet vorgesehen sind, wobei
eine Breitenabmessung und/oder eine Dickenabmessung von mindestens einer der mehreren Leiterbahnen (25) auf dem konischen Abschnitt (23) verändert ist, und
in dem großdurchmessrigen Abschnitt (21) und dem kleindurchmessrigen Abschnitt (22) eine Längserstreckung von mindestens einer der mehreren Leiterbahnen (25) einen Querschnittsbereich aufweist, der sich von dem der anderen Leiterbahnen in einer Querschnittsansicht des Führungsdrahtkerns (20) unterscheidet.

2. Der Führungsdraht (10) nach Anspruch 1, wobei
mindestens eine der mehreren Leiterbahnen (25) in der Querschnittsansicht des Führungsdrahtkerns (20) an verschiedenen Positionen in Längsrichtung eine von den anderen Leiterbahnen verschiedene Dickenabmessung aufweist.

3. Der Führungsdraht (10) nach Anspruch 1, wobei
mindestens eine der mehreren Leiterbahnen (25) in der Querschnittsansicht des Führungsdrahtkerns (20) an verschiedenen Positionen in Längsrichtung eine von den anderen Leiterbahnen verschiedene Breitenabmessung aufweist.

4. Der Führungsdraht (10) nach Anspruch 1, wobei
unter den Abständen (27) zwischen den mehreren Leiterbahnen (25) die Abstände am kleindurchmessrigen Abschnitt des Führungsdrahtkerns (20) größere Breitenabmessungen aufweisen als die Abstände am großdurchmessrigen Abschnitt des Führungsdrahtkerns (20) in der Querschnittsansicht des Führungsdrahtkerns (20).

5. Der Führungsdraht (10) nach Anspruch 1, wobei
unter den Abständen zwischen den mehreren Leiterbahnen (25) die Abstände am kleindurchmessrigen Abschnitt des Führungsdrahtkerns (20) kleinere Breitenabmessungen aufweisen als die Abstände am großdurchmessrigen Abschnitt des Führungsdrahtkerns (20) in der Querschnittsansicht des Führungsdrahtkerns (20).

6. Der Führungsdraht (10) nach Anspruch 1, wobei
mindestens eine der mehreren Leiterbahnen in der Querschnittsansicht des Führungsdrahtkerns (20) an verschiedenen Positionen in Längsrichtung eine veränderte Form aufweist.

7. Der Führungsdraht (10) nach Anspruch 6, wobei
eine Breitenabmessung von mindestens einer der mehreren Leiterbahnen (25) am kleindurchmessrigen Abschnitt (22) in der Querschnittsansicht des Führungsdrahtkerns (20) an verschiedenen Positionen in Längsrichtung verändert ist.

8. Der Führungsdraht (10) nach Anspruch 6, wobei
eine Dickenabmessung von mindestens einer der mehreren Leiterbahnen (25) am kleindurchmessrigen Abschnitt (22) in der Querschnittsansicht des Führungsdrahtkerns (20) verändert ist.

9. Der Führungsdraht (10) nach Anspruch 1, wobei
jede der mehreren Leiterbahnen (25) jeweils einen elektrischen Anschlussabschnitt (261-1, 261-2) aufweist und die mehreren elektrischen Anschlussabschnitte (261-1, 261-2) auf einer Geraden des Führungsdrahtkerns (20) angeordnet sind.

10. Der Führungsdraht (10) nach Anspruch 1, umfassend
eine zweite Isolierschicht (26), die die erste Isolierschicht (24) und die mehreren Leiterbahnen (25) abdeckt, wobei
die erste Isolierschicht (24) aus einem Material besteht, das eine niedrigere Dielektrizitätskonstante aufweist als die zweite Isolierschicht (26).

11. Der Führungsdraht (10) nach Anspruch 1, umfassend
eine zweite Isolierschicht (26), die die erste Isolierschicht (24) und die mehreren Leiterbahnen (25) abdeckt, wobei
die zweite Isolierschicht (26) aus einem Material besteht, das eine höhere Feuchtigkeitsbeständigkeit aufweist als die erste Isolierschicht (24).

12. Der Führungsdraht (10) nach Anspruch 1, umfassend
eine zweite Isolierschicht (26), die die erste Isolierschicht (24) und die mehreren Leiterbahnen (25) abdeckt, und
eine leitfähige Schicht, die auf einer Oberfläche der zweiten Isolierschicht (26) vorgesehen ist, wobei
die leitfähige Schicht elektrisch mit mindestens einer der mehreren Leiterbahnen verbunden ist.

13. Der Führungsdraht (10) nach Anspruch 1, umfassend
eine zweite Isolierschicht (26), die die erste Isolierschicht (24) und die mehreren Leiterbahnen (25) abdeckt, und
eine leitfähige Schicht, die auf einer Oberfläche der zweiten Isolierschicht (26) vorgesehen ist, wobei ein Teil der leitfähigen Schicht elektrisch mit dem Führungsdrahtkern (20) verbunden ist.

## Revendications

1. Un fil-guide (10) comprenant :
un noyau de fil-guide (20) comprenant :
une partie de grand diamètre (21),
une partie de petit diamètre (22) située distalement par rapport à la partie de grand diamètre (21) et ayant une dimension extérieure plus petite que celle de la partie de grand diamètre (21), et une partie conique (23) disposée entre la partie de grand diamètre (21) et la partie de petit diamètre (22),
une première couche isolante (24) prévue sur une surface du noyau de fil-guide (20), et
une pluralité de pistes conductrices (25) disposées à intervalles les unes des autres dans la direction circonférentielle du noyau de fil-guide (20) sur une surface de la première couche isolante (24), dans laquelle
une dimension de largeur et/ou une dimension d'épaisseur d'au moins une desdites pistes conductrices (25) varie sur la partie conique (23), et
dans la partie de grand diamètre (21) et la partie de petit diamètre (22), une extension longitudinale d'au moins une desdites pistes conductrices (25) présente une surface de section transversale différente de celles des extensions longitudinales des autres pistes conductrices dans une vue en section transversale du noyau de fil-guide (20).

2. Le fil-guide (10) selon la revendication 1, dans lequel
au moins une desdites pistes conductrices (25) présente, dans la vue en section transversale du noyau de fil-guide (20) à différentes positions dans la direction longitudinale, une dimension d'épaisseur différente de celles des autres pistes conductrices.

3. Le fil-guide (10) selon la revendication 1, dans lequel
au moins une desdites pistes conductrices (25) présente, dans la vue en section transversale du noyau de fil-guide (20) à différentes positions dans la direction longitudinale, une dimension de largeur différente de celles des autres pistes conductrices.

4. Le fil-guide (10) selon la revendication 1, dans lequel
parmi les espaces (27) entre lesdites pistes conductrices (25), les espaces de la partie de petit diamètre du noyau de fil-guide (20) ont des dimensions de largeur supérieures à celles des espaces de la partie de grand diamètre du noyau de fil-guide (20) dans la vue en section transversale du noyau de fil-guide (20).

5. Le fil-guide (10) selon la revendication 1, dans lequel
parmi les espaces entre lesdites pistes conductrices (25), les espaces de la partie de petit diamètre du noyau de fil-guide (20) ont des dimensions de largeur inférieures à celles des espaces de la partie de grand diamètre du noyau de fil-guide (20) dans la vue en section transversale du noyau de fil-guide (20).

6. Le fil-guide (10) selon la revendication 1, dans lequel
au moins une desdites pistes conductrices présente une forme modifiée dans la vue en section transversale du noyau de fil-guide (20) à différentes positions dans la direction longitudinale.

7. Le fil-guide (10) selon la revendication 6, dans lequel
une dimension de largeur d'au moins une desdites pistes conductrices (25) varie sur la partie de petit diamètre (22) dans la vue en section transversale du noyau de fil-guide (20) à différentes positions dans la direction longitudinale.

8. Le fil-guide (10) selon la revendication 6, dans lequel
une dimension d'épaisseur d'au moins une desdites pistes conductrices (25) varie sur la partie de petit diamètre (22) dans la vue en section transversale du noyau de fil-guide (20).

9. Le fil-guide (10) selon la revendication 1, dans lequel
chacune desdites pistes conductrices (25) présente respectivement une partie de connexion électrique (261-1, 261-2), et lesdites parties de connexion électrique multiples (261-1, 261-2) sont disposées sur une même ligne droite du noyau de fil-guide (20).

10. Le fil-guide (10) selon la revendication 1, comprenant
une deuxième couche isolante (26) recouvrant la première couche isolante (24) et lesdites pistes conductrices (25), dans lequel
la première couche isolante (24) est constituée d'un matériau ayant une constante diélectrique inférieure à celle de la deuxième couche isolante (26).

11. Le fil-guide (10) selon la revendication 1, comprenant
une deuxième couche isolante (26) recouvrant la première couche isolante (24) et lesdites pistes conductrices (25), dans lequel
la deuxième couche isolante (26) est constituée d'un matériau ayant une résistance à l'humidité supérieure à celle de la première couche isolante (24).

12. Le fil-guide (10) selon la revendication 1, comprenant
une deuxième couche isolante (26) recouvrant la première couche isolante (24) et lesdites pistes conductrices (25), et
une couche conductrice prévue sur une surface de la deuxième couche isolante (26), dans lequel
ladite couche conductrice est électriquement reliée à au moins une desdites pistes conductrices.

13. Le fil-guide (10) selon la revendication 1, comprenant
une deuxième couche isolante (26) recouvrant la première couche isolante (24) et lesdites pistes conductrices (25), et
une couche conductrice prévue sur une surface de la deuxième couche isolante (26), dans lequel une partie de ladite couche conductrice est électriquement reliée au noyau de fil-guide (20).
